# EUROPEAN PATENT SPECIFICATION

(11) **EP 4 065 197 B1**
(45) Date of publication and mention of the grant of the patent: **16.04.2025**
(21) Application number: 20804545.0
(22) Date of filing: 11.11.2020
(51) Int. Cl.: A61M 15/00, A61M 16/06, A61M 16/00, A61B 5/087

(54) **DEVICE WITH FLOW RATE INDICATOR**
VORRICHTUNG MIT DURCHFLUSSRATENANZEIGER
DISPOSITIF AVEC INDICATEUR DE DÉBIT

(30) Priority: 29.11.2019 GB 201917420; 05.05.2020 GB 202006647
(43) Date of publication of application: 05.10.2022
(73) Proprietor: Flexicare (Group) Limited, Mountain Ash, Mid Glamorgan CF45 4ER (GB)
(72) Inventor: THOMASON, Alexander James, Edinburgh Way Harlow Essex CM20 2TT (GB)
(74) Representative: Mewburn Ellis LLP
(86) International application number: PCT/EP2020/081803
(87) International publication number: WO 2021/104880

(56) References cited:
- EP-A1- 1 407 794
- EP-B1- 2 560 720
- WO-A1-2017/049034
- DE-A1- 2 803 993

## Description

### Field of the Disclosure

The present disclosure relates to a device for indicating a predetermined fluid flow rate, and in particular a device for indicating a predetermined air flow rate during user inhalation. In particular, the present disclosure relates to respiratory devices and inhaler devices, and more particularly drug delivery inhaler devices, such as pressurised metered dose inhaler (pMDI) devices and dry powder inhaler (DPI) devices. The disclosure also relates to methods of operation of such devices.

### Background

Pressurised metered dose inhaler (pMDI) devices are the most popular and widely prescribed devices for respiratory drug delivery, with approximately 500 million manufactured each year.

In such devices, the active ingredient/drug is typically provided in the form of a solution or suspension held in a pressurised canister. Actuation of the canister is typically achieved by depressing the canister towards a main body of the device. This causes an interaction between the canister valve and a valve seat/stem block that causes a metered dose to be ejected from the valve, along with a propellant gas (typically a hydrofluoroalkane (HFA) gas). The dose becomes aerosolised and available for inhalation by the patient.

Dry powder inhaler (DPI) devices are an alternative to these aerosol-based inhalers in which a powdered respiratory drug may be held within a capsule, for example, which is perforated by the device as the patient inhales. Alternative drug formats include blisters and reservoirs. An insufficient inhalation rate will result in reduced dose delivery and incomplete de-aggregation of the powdered drug, potentially leading to poor control of the respiratory problem.

Whilst it may appear that these devices are easy to use, this is not the case: seemingly simple steps such as coordination of actuation of the device with inhalation, and inhaling at the appropriate flow rate are often performed incorrectly. This misuse of drug delivery inhaler devices can lead to drug wastage and ineffective treatment.

GB-A-2372704 discloses a device for providing an indication of the respiratory flow rate of a patient. The device includes two reeds adapted to generate an audible signal at different air flow speeds through the device. The first reed generates an audible signal of a first pitch when the air flow reaches a predetermined minimum and the second reed generates an audible signal of a second pitch when the air flow reaches a predetermined maximum. Thus, the patient is informed when the air flow is within a desirable range, between a predetermined minimum and maximum.

GB-A-2490770 discloses an adaptor that may be fitted to a pMDI inhaler that incorporates an air flow rate indicator comprising a reed which oscillates and generates a sound signal at a predetermined minimum level suitable for delivery of the drug to the patient.

It is known that pMDls, for example, can differ in internal resistance. Examples include the relatively high resistance Atrovent^{®} pMDI and the low resistance Flutiform^{®} pMDI. When the patient inhales, air is drawn through the device via channels surrounding the canister in the pMDI. It is easier for air to passage through a low resistance inhaler than through a high resistance inhaler. DPI devices function via turbulent de-aggregation of the powder formulation. The turbulence property is differently sized within each device depending on its technical design. There are high, medium, and low resistance DPI devices. Patients, who may be using multiple inhaler types and drug formualations, are advised and tutored to inhale through their pMDI between approximately 20 - 60 L/min inspiratory flow rate, and through their DPI device at a much higher rate, approximately 90 L/min inspiratory flow rate. Inspiratory flow can also vary with the patient's ability, often dictated by disease severity. It is possible to anticipate clinical situations in which patients are both thoroughly confused and not capable of reproducing the ideal inspiratory flow rate for each device they may be required to use to control their disease.

The following inhalers for acoustically indicating a fluid flow rate through the device are also known, namely DE 28 03 993 A1, EP 1 407 794 A1, WO 2017/049034 A1 and EP 2 560 720 B1.

There is a desire to provide an improved air flow rate indicator for such devices (e.g. drug delivery inhaler devices including pMDIs and DPIs) that has a simple construction and improves a user's inhaling technique, to thus reduce drug wastage and provide effective treatment.

### Summary

The invention is defined in independent claim 1. Preferred embodiments are disclosed in the dependent claims.

In a first aspect, there is provided an inhaler device comprising: an open tube whistle; and a mouthpiece in fluid communication with an external opening of the inhaler device and a first end of the open tube whistle, wherein an inner diameter of the open tube whistle has a stepped profile such that, upon user inhalation through the mouthpiece, an audible/sound signal is generated at a predetermined fluid flow rate through the open tube whistle.

When a user inhales on the mouthpiece, air is drawn through both the external opening, and the open tube whistle. The stepped profile of the inner diameter of the open tube whistle acts as an acoustic impedance, such that, when the inhalation technique of the user is correct and the predetermined fluid flow rate is generated, an audible/sound signal is generated. The inventors have found that by providing the stepped profile of the inner diameter of the open tube whistle, a clear audible/sound signal is produced only when the fluid flow rate through the whistle is within a predetermined range of fluid flow rates. Thus, if the fluid flow rate through the whistle is too low, or too high, no (or a poor) audible/sound signal is produced.

The audible/sound signal provides feedback to the user that they have the correct inhalation technique. This helps to reduce drug wastage and provides effective treatment for the user/patient. Furthermore, the whistle provides immediate feedback to the user, thus efficiently teaching the user a correct inhalation technique.

Optional features will now be set out.

As used herein, the term "open tube whistle" is to be understood as a whistle comprising a tube (e.g. a cylinder) which is open at both ends.

As used herein, the term audible signal is to be understood as a sound signal audible to (e.g. capable of being detected or heard by) a human and/or a computing device. In other words, an audible signal may be a sound signal including frequencies that can be detected/heard by a human and/or frequencies that can be detected by a computing device having a sound receiver.

The stepped profile of the inner diameter of the open tube whistle is to be understood such that that the inner diameter of the open tube whistle changes in a step-wise manner along its length. For example, an inner diameter of a first portion of the open tube whistle may be greater than an inner diameter of a second portion of the open tube whistle with a stepped transition therebetween. The stepped transition is considered to be a discrete transition, or reveal, between the first portion of the open tube whistle and the second portion of the open tube whistle. The audible/sound signal may be generated only when the fluid flow rate through the open tube whistle is within a predetermined range of fluid flow rates.

A second end of the open tube whistle may provide an additional external opening of the inhaler device. The second end of the open tube whistle may be at an opposing end of the open tube whistle to the first end of the open tube whistle.

In this way, the inhaler device may comprise a first fluid flow path extending between the mouthpiece and the external opening of the inhaler device, and a second fluid flow path extending between the mouthpiece and the second end of the open tube whistle.

The second fluid flow path through the open tube whistle may be separate and distinct from the first fluid flow path.

Air may be drawn to the mouthpiece via the external opening, and also via the additional external opening and therefore through the open tube whistle. When a user inhales through the mouthpiece, more air may flow through the inhaler device via the external opening than via the additional external opening (and therefore via the open tube whistle). For example, 15-20% of the total air flow through the inhaler device may flow via the additional external opening (and therefore via the open tube whistle) in a single inhalation effort. More particularly, 17-18%, and more particularly approximately 17.9% of the total air flow through the inhaler device may flow via the additional external opening (and therefore via the open tube whistle).

Including the additional external opening in the inhaler device lowers the resistance (and therefore pressure drop) across the inhaler device because more air is able to flow through the device. The effect of the open tube whistle on inhaler device resistance may be dependent on the location of the open tube whistle, air flow bypass (i.e. first fluid flow path) and leakage rates (for example, via a dose counter of the inhaler device) of the first fluid flow path, and open tube whistle parameters.

In these embodiments, a uniform air resistance, and pressure drop, through the open tube whistle may be provided in any inhaler device which is designed to include the open tube whistle which sounds at a particular flow rate (e.g. designed with specific air flow bypass and leakage rates). Therefore, a uniform audible/sound signal is generated in any such inhaler device, which may help the user to establish whether or not they have the correct inhalation technique.

The open tube whistle may be completely internally embedded within the inhaler device. In these embodiments, a second end of the open tube whistle does not form an additional external opening. Instead, the second end of the open tube whistle may be in fluid communication with the external opening of the inhaler device. More particularly, there may be only one external opening in fluid communication with the mouthpiece in the inhaler device.

The second end of the open tube whistle (which may provide an additional external opening of the inhaler device), the first end of the open tube whistle, and an opening of the mouthpiece for communication with the mouth of the user may be substantially collinear. In this way, the second end of the open tube whistle and the mouthpiece may be on opposing sides of the inhaler device.

In some embodiments, the stepped profile of the inner diameter of the open tube whistle may comprise only one step. This may provide a simple sound generating mechanism which is easy to manufacture. Alternatively, the stepped profile of the inner diameter may comprise more than one step i.e. a plurality of steps such as two or more steps, three or more steps, four or more steps etc. Each step may have a same or similar height (e.g. rise) and/or width (e.g. run, going, or tread). Alternatively, the steps may have different heights and/or widths. The height and width of the steps may be chosen to generate sound with a specific (e.g. preferred) frequency.

In embodiments in which the stepped profile comprises only one step, the step may be closer to the second end of the open tube whistle, than the first end of the open tube whistle.

The inner diameter of a second portion of the open tube whistle adjacent to the second end of the open tube whistle (e.g. adjacent to the additional external opening) may be less than the inner diameter of a first portion of the open tube whistle adjacent to the first end of the open tube whistle.

The outer cross-sectional profile of the open tube whistle may be substantially circular, oval, barrel-shaped, or any other shape. The outer diameter of the open tube whistle may be substantially constant along its length. The inner cross-sectional profile of the open tube whistle may be substantially circular, oval or barrel-shaped.

Optionally, the open tube whistle may have an axial length of between 5 and 50mm, preferably between 10 and 20mm, more preferably between 14 and 18mm. The inventors have found that if the axial length of the open tube whistle is increased, the frequency of the generated audible/sound signal will be lower, and conversely, if the axial length of the open tube whistle is decreased, the frequency of the generated audible/sound signal will be higher. Furthermore, if the rate of air flow through the open tube whistle increases or decreases, the frequency of the generated audible/sound signal will naturally become higher or lower, respectively.

The second portion of the open tube whistle having a smaller inner diameter may have an inner diameter of between 0.5 and 5 mm, preferably between 1 and 3mm, more preferably between 1.5 and 2.5mm. The first portion of the open tube whistle having the larger inner diameter may have an inner diameter of between 1 and 5mm, preferably between 2 and 4mm, more preferably between 2.5 and 3.5mm. The ratio between the inner diameter of the second portion and the first portion may be between 1:1.4 and 1:1.8, and preferably between 1:1.5 and 1:1.6. The difference between the inner diameter of the first portion and second portion of the open tube whistle may be approximately 0.5mm, for example.

The axial length of the second portion having the smaller inner diameter may be less than the axial length of the first portion having the larger inner diameter. The ratio of the axial length of the second portion to the axial length of the entire open tube whistle may be between 1:5 and 1:20, for example. The ratio of the axial length of the second portion to the axial length of the entire open tube whistle may be between 1:10 and 1:12.

The second portion of the open tube whistle having the smaller inner diameter may have an axial length between 0.2mm and 4mm, more preferably, 0.5mm and 1.5mm, and more preferably less than 1mm. The inventors have found that providing the second portion of the open tube whistle having the smaller diameter with an axial length of less than 1mm provides a loud and clear audible/sound signal.

Optionally, a side wall of the open tube whistle may define at least one lateral hole. The inventors have found that providing a lateral hole in the side wall of the open tube whistle acts to shorten the length of the open tube whistle, and that introducing a lateral hole in the side wall results in a higher frequency generated audible/sound signal.

The lateral hole may be provided in the first portion of the open tube whistle having the larger inner diameter. The inventors have found that moving the lateral hole from a position closer to the first end of the open tube whistle to a position closer to the second end of the open tube whistle results in an increase in frequency of generated audible/sound signal (i.e. being higher). In other words, when the lateral hole is provided closer to the step, the frequency of the generated audible/sound signal is higher than if the lateral hole is provided further from the step.

By changing the parameters of the open tube whistle (e.g. axial length, ratio of first and second portions, positioning of lateral holes etc.), resistance effects of the open tube whistle are altered and so the sound generated by the open tube whistle is altered. Different inhaler devices for different medications, or treatments could incorporate open tube whistles with different parameters, to thus help a user to differentiate between inhaler devices (and therefore different medications/treatments). However, the resistance of the open tube whistle may largely still be controlled by the air flow bypass rate through the first fluid flow path (and therefore via the external opening).

The inhaler device is preferably adapted such that the audible/sound signal is generated at an air flow rate of between 20 and 200 L/min, and more preferably between 20 and 90 L/min.

In some embodiments, the inhaler device may further comprise a stem block for location of a drug reservoir (e.g. canister), the drug reservoir being operable to deliver a dose of drug through a drug delivery output aperture into the mouthpiece for inhalation by the user. The stem block may act as a seat for location of the drug reservoir. The stem block may ensure a good fit with a canister valve stem in fluid communication with the drug reservoir such that leakage or failure does not occur. The stem block may differ in geometry to accommodate different canister valve stem types or formulation parameters, for example. The drug delivery output aperture (e.g. spray hole) may be included in the stem block, and may vary in diameter and length according to aerosol spray characteristics.

Optionally, the open tube whistle may extend through the stem block to be in fluid communication with the mouthpiece.

The drug delivery output aperture may be separate, distinct, and spaced from the first end of the open tube whistle.

The inhaler may comprise an upright main body for housing the drug reservoir. The mouthpiece may extend transversely from a lower end of the upright main body. The upright main body and the mouthpiece may be integrally formed.

The second end of the open tube whistle (and therefore the additional external opening of the inhaler device) and the mouthpiece may be on opposing lateral sides of the upright main body.

One or more balance protrusions may extend from the lower end of the upright main body and/or the mouthpiece. In some embodiments, one or more balance protrusions may extend from a bottom surface of the upright main body, wherein the bottom surface is at the lower end of the upright main body. The balance protrusion(s) may stabilise the inhaler device when the inhaler device is placed on a flat surface, by providing multiple points of contact with the flat surface. In some embodiments, multiple points of contact with a flat surface may be provided by the mouthpiece and at least two (e.g. a pair of) balance protrusions extending from the bottom surface of the upright main body, and this may stabilise the inhaler device when the bottom surface of the upright main body is placed on the flat surface (i.e. when the inhaler device is standing upright).

Optionally, the upright main body, the stem block and the open tube whistle may be integrally formed. For example, the upright main body, stem block and open tube whistle may be injection moulded as a single part. In this way, no assembly or disassembly of the inhaler would be required by the user, and the device would be simple to use.

In further embodiments, the upright main body, stem block, open tube whistle and mouthpiece may be integrally formed (e.g. injection moulded as a single part). In alternative embodiments the mouthpiece may be formed separately and thus reversibly detachable from/attachable to the integrally formed stem block, upright main body and open tube whistle.

In other embodiments, the open tube whistle may be reversibly and slidably receivable in the inhaler device. For example, the open tube whistle may be reversibly and slidably receivable in the upright main body. The open tube whistle may be reversibly and slidably receivable in the stem block. In these embodiments, the open tube whistle and the stem block may be formed as separate components of the inhaler device.

In other embodiments, the stem block and the open tube whistle may be integrally formed. For example, the stem block and the open tube whistle may be injection moulded as a single part. The single-part stem block and open tube whistle may be reversibly and slidably receivable in the inhaler device. Specifically, the single-part stem block and open tube whistle may be reversibly and slidably receivable in the upright main body (which may or may not be integral with the mouthpiece). In this way, a user may assemble/disassemble their own inhaler device. By providing the single-part stem block and open tube whistle separately from the remaining parts of the inhaler device (e.g. the upright main body and mouthpiece), some components of the device can be reused. Accordingly, the inhaler device has improved sustainability, by improving the reusability of many components of the inhaler device, and reducing the burden on recycling and landfill.

The single-part stem block and open tube whistle may alternatively be irreversibly fixed within the inhaler device, and specifically within the upright-main body (e.g. during manufacture). In this way, a user is not required to assemble their own inhaler device, thus making use of the device simpler.

Optionally, the open tube whistle, stem block and upright main body may be formed as separate components of the inhaler device. Optionally, the open tube whistle, stem block, upright main body and mouthpiece may all be formed as separate components of the inhaler device.

By providing the components of the inhaler device separately, any contaminated (e.g. drug-contaminated) components of the inhaler device (such as the stem block and the drug reservoir) can be removed and disposed of, whereas other components of the inhaler device (such as the open tube whistle, the upright main body and the mouthpiece) can be reused. Accordingly, the inhaler device has improved sustainability, by improving the reusability of many components of the inhaler device, and reducing the burden on recycling and landfill.

When the open tube whistle, stem block and upright main body (and optionally the mouthpiece) are formed as separate components of the inhaler device, the stem block may be reversibly and slidably receivable in the upright main body, and the open tube whistle may extend through the stem block to lock the stem block to the upright main body.

Accordingly, in these embodiments, not only does the open tube whistle provide audible feedback to a user to indicate when the user is inhaling correctly, the open tube whistle also acts as a locking mechanism to lock the stem block to the upright main body. The open tube whistle can therefore be incorporated as a sliding locking component to engage and disengage the removable stem block from the upright main body. This helps to improve sustainability.

Furthermore, stem blocks can often be prone to shrinkage during injection moulding/manufacturing process due to material thickness. Deformation of the stem block may occur for this or other reasons. In order to address this problem, it is known to include a cut-out below the drug delivery output aperture, which is formed purely to prevent this deformation. However, by providing the open tube whistle, stem block and upright main body as separate components, and by using the open tube whistle as a locking mechanism to lock the open tube whistle to the upright main body, this cut-out in the stem block is no longer required. This is because the open tube whistle, stem block and upright main body are not manufactured in a single injection moulding process, and so the stem block of the present disclosure is no longer prone to shrinkage during manufacture. This helps to improve manufacturability, quality and appearance of the inhaler device.

A counter may be arranged within the upright main body and the open tube whistle may extend through the counter for fluid communication with the mouthpiece. The counter may count the number of user actuations of the inhaler device.

The open tube whistle and counter may be integrally formed.

Alternatively, the counter, open tube whistle, upright main body, and optionally stem block, may be formed as separate components. In these embodiments, the open tube whistle may be reversibly and slidably receivable in the counter. Removing the open tube whistle from the counter may automatically reset the counter.

The counter may be reversibly and slidably receivable in the upright main body, and the open tube whistle may extend through the counter to lock the counter to the upright main body. The open tube whistle therefore also acts as a locking mechanism to lock the counter to the upright main body. The open tube whistle can therefore be incorporated as a sliding locking component to engage and disengage the removable counter from the upright main body. This further helps to improve sustainability.

Optionally, the inhaler device may further comprise a magnifying lens. The magnifying lens may be arranged to enhance the visibility of the counter, and specifically a number of user actuations of the inhaler device indicated by the counter. In other words, the magnifying lens may magnify the number of user actuations of the inhaler device indicated or displayed by the counter, for improved visibility.

The stem block, open tube whistle and magnifying lens may be integrally formed. Specifically, the stem block, open tube whistle and magnifying lens may be formed (e.g. injection moulded) as a single part. This single-part stem block, open tube whistle and magnifying lens may be reversibly and slidably receivable in the inhaler device, and specifically in the upright main body. It may be secured in the upright main body by an interference or snap fit connection. It may further comprise a pair of assembly tabs to aid the insertion and removal of the single-part stem block, open tube whistle and magnifying lens in the upright main body. In this way, a user may assemble/disassemble their own inhaler device. By providing the single-part stem block, open tube whistle and magnifying lens separately from the remaining parts of the inhaler device (e.g. the upright main body and mouthpiece), some components of the device can be reused. Accordingly, the inhaler device has improved sustainability, by improving the reusability of many components of the inhaler device, and reducing the burden on recycling and landfill.

The single-part stem block, open tube whistle and magnifying lens may alternatively be irreversible fixed within the inhaler device, and specifically within the upright-main body (e.g. during manufacture). In this way, a user is not required to assemble their own inhaler device, thus making use of the device simpler.

Alternatively, the stem block, open tube whistle and magnifying lens may be formed as separate components. In further alternative embodiments, the stem block may be formed as a separate component from the open tube whistle and magnifying lens which may be formed as a single component.

The magnifying lens may be formed from a transparent polymer material. When the stem block, open tube whistle and magnifying lens are formed as a single integral part, this single integral part may be formed from a transparent polymer material.

Optionally, the upright main body may comprise a locating feature for correctly positioning the stem block in the upright main body. The stem block may also comprise a locating feature which complements the locating feature on the upright main body. These complementary locating features on the upright main body and the stem block may correspond (e.g. interlock) with each other to thereby correctly position and align the stem block within the upright main body.

The stem block locating feature may be a locating protrusion and the main body locating feature may be a locating recess, the stem block locating protrusion being configured to interlock with the main body locating recess. Alternatively, the stem block locating feature may be a locating recess and the main body locating feature may be a locating protrusion, the main body locating protrusion being configured to interlock with the stem block locating recess.

In some embodiments, the upright main body may comprise a stem block receiving portion for receiving the stem block, the stem block receiving portion comprising the main body locating features. The stem block may be retained in the stem block receiving portion by a push-fit fitting.

Optionally, the inhaler device may comprise a stem block guider for guiding the stem block along one or more protruding ribs in the upright main body. The one or more protruding ribs in the upright main body may act as a guide rail to guide the stem block into the stem block receiving portion in the correct orientation and position. The stem block guider and the one or more ribs may have a sliding fit.

The stem block guider may be integrally formed with the stem block (i.e. as a single component). Alternatively, the stem block guider and the stem block may be formed as two separate and distinct components, which may interlock, or be attachable to one another.

Optionally, the open tube whistle may comprise a handle tab. This may help a user to slidably insert and remove the open tube whistle in the stem block. The handle tab may be adjacent to the second end of the open tube whistle, for example.

The open tube whistle may be biased into an assembled position in which the open tube whistle extends through the stem block. The open tube whistle may be biased into the assembled position by a snap-fit mechanism. Specifically, the open tube whistle may comprise a projection for biasing the open tube whistle into the assembled position. Alternatively/additionally, the open tube whistle may be biased into the assembled position by a spring mechanism.

The open tube whistle, the stem block, the mouthpiece and/or the upright main body may be formed of plastic.

The inhaler device may further comprise the drug reservoir. The external opening of the inhaler device may completely encircle the drug reservoir. When a user inhales, more air may flow through the inhaler device via the external opening of the inhaler device than via the additional opening of the inhaler device (and therefore via the open tube whistle).

The inhaler device may comprise a plurality (e.g. 2, 3 or more) open tube whistles, wherein the plurality of open tube whistles may have different parameters to one another. In this way, the plurality of open tube whistles may generate audible/sound signals of different pitches/frequencies at different predetermined fluid flow rates through the respective open tube whistles. These audible/sound signals of different pitches/frequencies may indicate an upper and lower limit of a preferred fluid flow rate range, for example.

The inhaler device may be a drug delivery inhaler device. For example, the inhaler device may be a pressurised metered dose inhaler (pMDI) device. Alternatively, the inhaler device may be a dry powder inhaler (DPI) device, a breath-actuated pressurised metered dose inhaler, a soft mist inhaler, or a spacer.

The drug reservoir may contain an active ingredient/drug, which may be provided in the form of a solution or suspension. The active ingredient may be salbutamol, which is marketed as Ventolin^{®} among other brand names. The drug reservoir may also contain a propellant formulation/gas, such as hydroflouroalkane (HFA) gas, in particular HFA152a, which is marketed as Zephex^{®} 152a. In particular, the inhaler device may be a drug delivery inhaler device, comprising a salbutamol/HFA152a formulation.

Optionally, the audible/sound signal generated by the open tube whistle may be detectable by software. The software may be configured to monitor, record and analyse the fluid flow rate through the open tube whistle and/or to monitor, record and analyse breathing patterns, and to determine whether a user is using the inhaler device correctly (i.e. using the device too little or too often, for example).

Furthermore, the software may be configured to detect frequencies and harmonics of the audible/sound signal generated by the open tube whistle(s) which occur at predetermined fluid flow rates. The inventors have found that the fundamental frequency of the audible/sound signal is controlled predominantly by the axial length of the open tube whistle and the fluid flow rate, whereas other open tube whistle parameters (such as step length, inner diameter etc.) determine the harmonics, whistle quality and duration of the audible/sound signal. Therefore, software may be configured to detect different shaped open tube whistles (which could indicate different inhaler devices used for different medications/treatments) based on the monitoring and analysis of frequencies and harmonics of the generated audible/sound signal. The software may also be configured to provide feedback to a user via an application on a mobile device, or a smart speaker, for example.

According to a second aspect, there is provided a system comprising an inhaler device according to the first aspect, and a computing device having a sound receiver for detecting the audible/sound signal.

The computing device may be a mobile device, such as a mobile phone, for example.

The system may further comprise computer software for running on the computing device. The computing device may be configured to monitor, record and/or analyse the fluid flow rate through the open tube whistle and/or breathing patterns based on the audible/sound signal detected by the sound receiver, for example by detecting frequencies and/or harmonics of the audible/sound signal.

According to a third aspect, there is provided a method of monitoring use of an inhaler device, the method comprising:
providing a system according to the second aspect; and
detecting the audible/sound signal generated when the fluid flow rate through the open tube whistle is at the predetermined fluid flow rate.

The method may further comprise recording (e.g. using computer software such as an application for running on a computing device) the duration, frequency and/or harmonics of the audible/sound signal. This information may be used to monitor use of the inhaler device by the user/patient. It can be used to ensure that the user had a correct inhalation technique.

The method may further comprise providing user feedback, via an application on a mobile device or a smart speaker, for example.

According to a fourth aspect, there is provided a kit of parts for indicating a desired fluid flow rate, the kit of parts comprising:
an open tube whistle; and
a stem block for an inhaler device, the stem block for locating a drug reservoir operable to deliver a dose of drug through a drug delivery output aperture in the stem block, wherein the open tube whistle is reversibly and slidably receivable through the stem block.

The open tube whistle may be as described above with respect to the first aspect. The stem block may be as described above with respect to any option of the first aspect.

Therefore, the open tube whistle may have a stepped profile such that an audible/sound signal is generated at a predetermined fluid flow rate through the open tube whistle.

The kit of parts may comprise the drug reservoir. The drug reservoir may contain an active ingredient/drug, which may be provided in the form of a solution or suspension. The active ingredient may be salbutamol, which is marketed as Ventolin^{®} among other brand names. The drug reservoir may also contain a propellant formulation/gas, such as hydroflouroalkane (HFA) gas, in particular HFA152a, which is marketed as Zephex^{®} 152a. The kit of parts may further comprise an upright main body for housing the drug reservoir and the stem block, and/or a mouthpiece.

The kit of parts may be configured to be assembled to form the inhaler device as described above with respect to the first aspect.

According to a fifth aspect, there is provided a method of using an inhaler device for indicating a desired fluid flow rate, the method comprising the steps of:
providing the kit of parts of the fourth aspect;
locating the stem block in a main body for housing the drug reservoir; and
positioning the open tube whistle in the stem block such that the open tube whistle extends through the stem block.

The inhaler device may be as described above with relation to the first aspect.

The method may further comprise using a locating feature on the main body for correctly positioning the stem block in the main body.

The step of locating the stem block in the main body may comprise receiving the stem block in a stem block receiving portion by interlocking a locating feature on the stem block with a locating feature on the stem block receiving portion.

Optionally, the method may further comprising using a stem block guider for guiding the stem block along one or more protruding ribs in the main body.

The method may further comprise the step of removing the open tube whistle from the stem block, and removing the stem lock from the main body by pushing the stem block from the main body via a hole in the main body.

According to a sixth aspect, there is provided an apparatus for indicating a desired fluid flow rate through an inhaler device, the apparatus comprising:
a stem block for locating a drug reservoir operable to deliver a dose of drug through a drug delivery output aperture in the stem block; and
an open tube whistle extending through the stem block, wherein the open tube whistle and stem block are integrally formed, and wherein the apparatus is configured to be reversibly and slidably receivable in an inhaler device.

The open tube whistle may be as described above with respect to the first aspect. Therefore the open tube whistle may have a stepped profile such that an audible/sound signal is generated at a predetermined fluid flow rather through the open tube whistle.

The stem block may be as described above with respect to the first aspect.

The apparatus may further comprise a magnifying lens. The magnifying lens may be as described above with respect to the first aspect. The stem block, open tube whistle and magnifying lens may be integrally formed.

According to a seventh aspect, there is provided a method of using an inhaler device for indicating a desire fluid flow rate, the method comprising the steps of:
providing the apparatus of the sixth aspect;
slidably receiving the apparatus in a main body of an inhaler device, the main body for housing the drug reservoir.

The inhaler device may be as described above with relation to the first aspect.

According to an eighth aspect, there is provided a system for monitoring breathing patterns of a user, the system comprising:
an open tube whistle, the inner diameter of the open tube whistle having a stepped profile, such that, upon user inhalation or exhalation through the open tube whistle, an audible/sound signal is generated at a predetermined fluid flow rate through the open tube whistle; and
a computing device having a sound receiver for detecting the audible/sound signal.

The open tube whistle may be as described above with relation to the first aspect. The computing device may be a mobile device, such as a mobile phone, for example.

The system may further comprise computer software for running on the computing device. The computing device may be configured to monitor, record and/or analyse the fluid flow rate through the open tube whistle and/or breathing patterns based on the audible/sound signal detected by the sound receiver.

The open tube whistle of the system of the eighth aspect may be incorporated into an inhaler device, such as that described above in relation to the first aspect, although the eighth aspect is not limited to inhaler devices. Instead, the system of the eighth aspect may be configured to monitor breathing patterns of babies (while awake or asleep); first responders in extreme environments (e.g. monitor for stress and/or overexertion); pilots, high altitude climbers, mountain rescue or the military (to detect early signs of altitude sickness), scuba divers (to detect early signs of lung function problems), mine workers (to detect early signs of tachycardia or hypoxia) or animals (to identify lung abnormalities). For example, the open tube whistle may be incorporated into an oral mask assembly, as discussed in relation to the tenth aspect below.

The computing device may be configured to monitor a frequency (e.g. pitch) of the audible/sound signal, a duration of the audible/sound signal and/or a number of audible/sound signals produced in a predetermined period of time (e.g. per minute). Based on this monitoring, the computing device may be configured to detect a lung function condition and/or a lung function abnormality, such as eupnoea (normal breathing rate and pattern), tachypnoea (increased respiratory rate), bradypnoea (decreased respiratory rate), apnoea (absence of breathing), hyperpnoea (increased depth and rate of breathing), Cheyne-Stokes condition (gradual increases and decreases in respirations with period of apnoea), Biot's condition (abnormal breathing patterns with clusters of rapid respiration of equal depth and regular apnoea periods), Kussmaul breathing (tachypnoea and hyperpnoea) and apneustic respiration (prolonged inspiratory phase with a prolonged expiratory phase). Based on such a detection, the computing device may be configured to provide feedback, such as a warning, to a user or a health care professional, e.g. via a display.

Accordingly, to a ninth aspect, there is provided a method of monitoring breathing patterns of a user, the method comprising:
providing a system according to the eighth aspect; and
detecting the audible/sound signal generated when the fluid flow rate through the open tube whistle is at the predetermined fluid flow rate.

The method may further comprise recording (e.g. using computer software such as an application for running on a computing device such as a mobile device) the duration and/or frequency of the audible/sound signal.

According to a tenth aspect, there is provided an oral mask assembly comprising a breathing mask for covering the mouth and nose of a user, and at least one open tube whistle incorporated into the breathing mask, wherein an inner diameter of the at least one open tube whistle has a stepped profile such that, upon user inhalation and/or exhalation when the breathing mask is covering the mouth and/or nose of a user, an audible/sound signal is generated at a predetermined fluid flow rate through the open tube whistle.

When a user is wearing the breathing mask such that the breathing mask is covering their mouth and/or nose, air is drawn through the open tube whistle. The stepped profile of the open tube whistle acts as an acoustic impedance, such that when a predetermined fluid flow rate is generated by the user inhalation and/or exhalation, an audible/sound signal is generated. As described above in relation to the first aspect, the inventors have found that by providing the stepped profile of the inner diameter of the open tube whistle, a clear audible/sound signal is produced only when the fluid flow rate through the whistle is within a predetermined range of fluid flow rates. Thus, if the fluid flow rate through the whistle is too low, or too high, no (or a poor) audible/sound signal is produced.

The incorporation of the open tube whistle into the breathing mask allows for monitoring of inspiratory and/or expiratory breathing patterns. In particular, the audible/sound signal may be audible to a human and/or a computing device, and thus the audible/sound signal frequency (e.g. pitch), the audible/sound signal duration and/or the number of audible/sound signals produced in a predefined period of time (e.g. per minute), may provide feedback to a user (directly, or via a computing device) to indicate a lung function condition and/or abnormality.

The open tube whistle may be as described above in relation to previous aspects.

The open tube whistle may be configured to generate an audible/sound signal at an (average) fluid flow rate of 6-8L/min. This audible/sound signal may indicate eupnoea (e.g. normal breathing rate and pattern). The open tube whistle may be configured to generate an audible/sound signal at an (average) fluid flow rate of >60L/min. This audible/sound signal may indicate tachypnoea (increased respiratory rate).

The open tube whistle may extend through the breathing mask, between an internal side of the breathing mask (e.g. adjacent to the user's mouth and nose, in use) and an external side of the breathing mask.

As described above in relation to previous aspects, an inner diameter of a first portion of the open tube whistle may be greater than an inner diameter of a second portion of the open tube whistle with a stepped transition therebetween. The first portion of the open tube whistle having the greater inner diameter may be in fluid communication with (e.g. adjacent to) the internal side of the breathing mask and the second portion of the open tube whistle having a smaller inner diameter may be in fluid communication with (e.g. adjacent to) the external side of the breathing mask. Alternatively, the first portion of the open tube whistle may be in fluid communication with (e.g. adjacent to) the external side of the breathing mask and the second portion of the open tube whistle may be in fluid communication with (e.g. adjacent to) the internal side of the breathing mask.

The oral mask assembly may comprise a plurality of (e.g. two) open tube whistles incorporated into the breathing mask. One of the open tube whistles may be for generating an audible/sound signal at a predetermined fluid flow rate through the open tube whistle upon user inhalation, and the other may be for generating an audible/sound signal at a predetermined fluid flow rate through the open tube whistle upon user exhalation.

The stepped profiles of the plurality of open tube whistles may have different dimensions and/or geometries, and therefore may generate an audible/sound signal at different predetermined fluid flow rates and/or frequencies to indicate different lung function conditions, for example. For example, a first open tube whistle may be configured to generate an audible/sound signal at a fluid flow rate of 6-8L/min (therefore indicating eupnoea), and a second open tube whistle may be configured to generate an audible/sound signal at a fluid flow rate of >60L/min (therefore indicating tachypnoea).

A main body of the breathing mask may be injection moulded from a polymer material such as silicone, polyvinyl chloride (PVC), acrylonitrile butadiene styrene (ABS), polypropylene (PP) etc. A rim of the main body of the breathing mask may be configured to provide a tight seal to the user around the user's nose and mouth, in use. For example the rim may comprise rubber, a plastic/polymer, silicone and/or a foam (e.g. Polyvinyl Chloride, PVC), and may be lip-shaped for providing a tight seal to the user's skin. The oral mask assembly may comprise strap attachment means, such as loop buckles, strap handles or strap slots, for attaching to a strap configured to strap the oral mask assembly to the user's face.

The breathing mask may comprise a pair of one-way valves. A first of the one-way valves may be for user inhalation and a second of the one-way valves may be for user exhalation. Accordingly, the first one-way valve may only permit fluid flow from the external side to the internal side of the breathing mask, whereas the second one-way valve may only permit fluid flow from the internal side of the breathing mask to the external side of the breathing mask. The one-way valves may be assembled into the breathing mask via a snap-fit connection and/or an interference fit.

Additionally/alternatively, the breathing mask may comprise one or more bypass channels extending through the breathing mask between the internal and external sides of the breathing mask. Each bypass channel may comprise a hole or a slot and may act as a fluid/air leak point or fluid/air bypass. The bypass channel(s) (in addition to the open tube whistle dimensions) may help to adjust/control the resistance of the fluid (e.g. air) flow between the internal and external sides of the breathing mask. In particular, a bypass channel having a larger inner diameter will provide less resistance, and therefore enable less restrictive breathing, than a bypass channel having a smaller inner diameter. The size/geometry of the bypass channel(s), in addition to the open tube whistle(s) geometries, impacts the predetermined fluid flow rate at which the audible/sound signal is generated. In particular, the larger the inner diameter of the bypass channel, the greater the predetermined fluid flow rate at which the open tube whistle(s) generates an audible/sound signal.

The breathing mask may comprise a plurality of bypass channels. Each bypass channel may have a same/similar geometry (e.g. inner diameter), or the bypass channels may have different geometries (e.g. different inner diameters). For example, one or more bypass channels may be formed in fluid communication with (e.g. in, at or adjacent to) a respective one-way valve, and the bypass channel(s) formed in fluid communication with the one-way valve for user inhalation may have a different geometry/size to the bypass channel(s) formed in, at or adjacent to the one-way valve for user exhalation. Therefore, the resistance, and thus the acoustic performance of the open tube whistle (e.g. the predetermined fluid flow rate), is controlled for inhalation and exhalation separately, and with improved accuracy.

The one or more open tube whistles, one or more filters, and/or the one-way valves may be reversibly attachable to the main body of the breathing mask. In this way, the main body be (re)usable with replacement open tube whistles, filters and/or one-way valves.

The oral mask assembly may comprise one or more open tube whistles integrally moulded within the main body and/or an add-on component attachable to the main body of the breathing mask, wherein one or more open tube whistles are incorporated in the add-on component. The one or more open tube whistles may be integrally moulded in the add-on component.

The add-on component may be attachable to the main body of the breathing mask via a threaded connection, or a snap-fit or push-fit connection. The add-on component may comprise one or more bypass channels and/or a pair of one-way valves, as described above.

The breathing mask and/or the add-on assembly may comprise a filter. The filter may be an antimicrobial medical grade filter, for example.

The audible/sound signal may be detectable by a computing device, as described above. Alternatively/additionally, the audible/sound signal may be detectable by a human ear. The disclosure includes the combination of the aspects and optional features described above except where such a combination is clearly impermissible or expressly avoided.

### Brief Description of the Drawings

Embodiments of the invention will now be described by way of example with reference to the accompanying drawings in which:
Figure 1 shows an exploded view of an inhaler device;
Figure 2a and 2b show perspective views of an open tube whistle for the inhaler device of Figure 1;
Figure 3a shows another perspective view of an open tube whistle for an inhaler device;
Figure 3b shows a longitudinal cross-sectional view of the open tube whistle of Figure 3a;
Figures 4a and 4b show perspective views of a stem block for an inhaler device;
Figures 5a-5d illustrate steps in a method for using an inhaler device having an open tube whistle;
Figures 6a-6b illustrate further steps in a method for using an inhaler device having an open tube whistle;
Figure 7 shows an exploded view of an inhaler device;
Figures 8a and 8b show perspective views of a stem block and stem block guider for an inhaler device;
Figures 9a-9c illustrate steps in a method for using an inhaler device having an open tube whistle, a stem block and a stem block guider;
Figures 10a-10b illustrate further steps in a method for using an inhaler device having an open tube whistle, a stem block and a stem block guider;
Figures 11a-11d show different perspective views of an inhaler device having an integrated open tube whistle;
Figures 12a-12b show perspective views of a stem block and stem block guider for an inhaler device;
Figure 13 shows an exploded view of an inhaler device;
Figures 14a-14c show different perspective views of an inhaler device having a mouthpiece cap;
Figures 15a-15b show different perspective views of another inhaler device having a mouthpiece cap;
Figures 15c-15d show different perspective views of a mouthpiece cap and an open tube whistle of the inhaler device of Figures 15a-15b;
Figures 16a-16c show different perspective views of an inhaler device having a mouthpiece cap;
Figures 17a-b show perspective views of inhaler devices having a counter;
Figures 18a-f show perspective views of inhaler devices having a magnifier for improving visibility of a counter;
Figure 19 shows a cross-sectional view of an inhaler device having a magnifier for improving visibility of a counter;
Figures 20a and 20b show perspective views of an upright main body of an inhaler device;
Figures 21a-21h show cross-sectional views of open tube whistles for an inhaler device;
Figures 22a-22h show exaggerated cross-sectional views of Figures 21a-21h respectively;
Figures 23a and 23b show cross-sectional views of open tube whistles having a plurality of steps;
Figure 24 shows a graph comparing the performance of an inhaler device comprising an open tube whistle with an inhaler device without an open tube whistle;
Figure 25 shows a graph comparing the performance of a pMDI comprising a salbutamol/HFA152a formulation and with an open tube whistle, with a pMDI comprising a salbutamol/HFA152a formulation without an open tube whistle;
Figures 26a-26d show different perspective and cross-sectional views of an oral mask assembly;
Figures 27a and 27b show a cross-sectional view of the oral mask assembly of Figures 26a-26d illustrating the flow of air through the oral mask as a user inhales and exhales, respectively;
Figures 28a-28c show different perspective and cross-sectional views of another oral mask assembly;
Figures 29a and 29b show different perspective views of an oral mask assembly with an add-on component; and
Figures 30a and 30b show exploded views of the oral mask assembly and add-on component of Figures 29a and 29b.

### Detailed Description and Further Optional Features

Aspects and embodiments will now be discussed with reference to the accompanying figures. Further aspects and embodiments will be apparent to those skilled in the art.

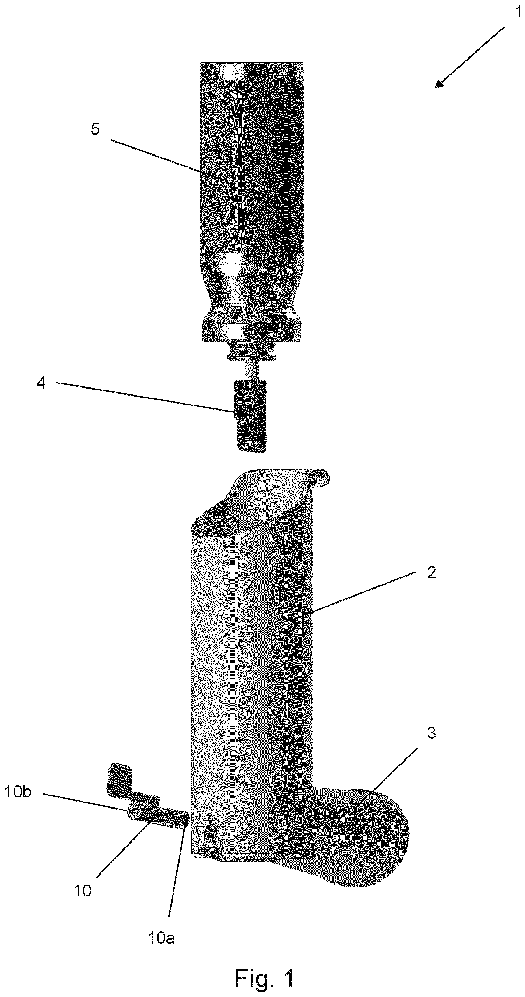

Figure 1 shows an exploded perspective view of a pressurised metered dose inhaler (pMDI) 1 adapted to deliver respiratory drugs to a patient. The pMDI comprises an upright main body 2 and a transverse mouthpiece 3 extending from a lower end of the upright main body 2. The mouthpiece 3 has an opening for communication with the mouth of a patient. The upright main body 2 is substantially cylindrical (with a substantially cylindrical transverse cross-section) and the transverse mouthpiece 3 has a substantially oval or barrel-shaped transverse cross-section so that the mouthpiece 3 can easily form a seal with the patient's mouth.

The pMDI 1 further comprises a stem block 4 for location of a drug canister 5 for containing a respiratory drug. Although not shown in Figure 1, in an assembled state, the stem block is positioned at the junction between the upright main body 2 and the transverse mouthpiece 3. In the assembled state, a canister 5 is inserted into the upright main body 2 and is housed in the upright main body 2. The canister 5 is pressurised and contains the respiratory drug in the form of a liquid in solution or suspension.

In the assembled state, the mouthpiece 3 is in fluid communication with an external opening of the pMDI 1 surrounding the canister 5.

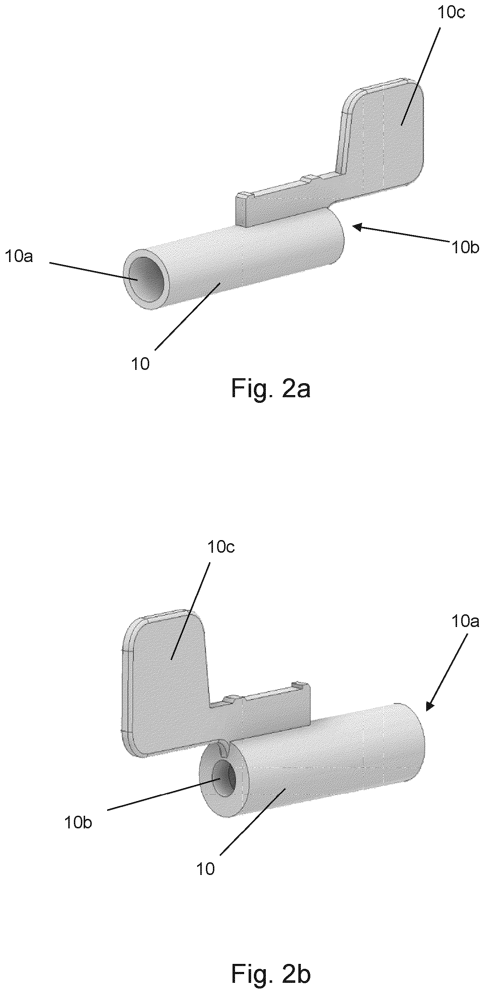

The pMDI 1 further comprises an open tube whistle 10. As shown in Figures 2a and 2b, the open tube whistle is a cylindrical tube which is open at both ends.

In the embodiment shown in Figure 1, the upright main body 2 and mouthpiece 3 are integrally formed as a single plastic component, and the stem block 4 and open tube whistle 10 are formed as separate components. Although not shown in Figure 1, the open tube whistle 10 is insertable into the upright main body 3 such that a first end 10a of the open tube whistle 10 is in fluid communication with the mouthpiece 3, and a second end 10b of the open tube whistle 10 forms an additional external opening of the pMDI 1. The second end 10b of the open tube whistle (which provides the additional external opening of the inhaler device 10) is on an opposing lateral side of the upright main body 2 to the mouthpiece 3. Specifically, the second end 10b of the open tube whistle 10, the first end 10a of the open tube whistle and the opening of the mouthpiece 3 are substantially collinear.

Accordingly, the open tube whistle 10 extends transversely (relative to the axial length of the upright main body) into the upright main body 2 of the pMDI 1.

The open tube whistle 10 shown in Figures 2a and 2b further comprises a handle tab 10c adjacent to the second end 10b of the open tube whistle 10. The handle tab 10c is discussed in further detail below.

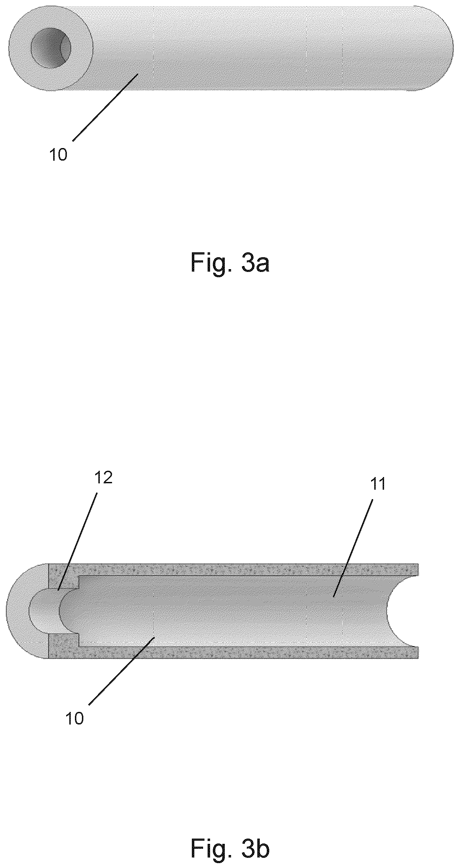

Figure 3a shows an open tube whistle 10 for an inhaler device, such as the pMDI 1 of Figure 1, without a handle tab. The outer cross-sectional profile of the open tube whistle 10 is substantially circular, and the outer diameter of the open tube whistle is substantially constant along its length. As previously mentioned, the open tube whistle 10 is open at both ends.

The open tube whistle may have an axial length of between 14 and 18mm.

Figure 3b is a cross-section of the open tube whistle 10 of Figure 3a, which illustrates the outer diameter and inner diameter of the open tube whistle 10. The inner cross-sectional profile of the open tube whistle 10 is substantially circular.

The inner diameter of the open tube whistle 10 has a stepped profile having a single step between a first portion 11 and a second portion 12. The first portion 11 of the open tube whistle 10 has a larger inner diameter than the second portion 12 of the open tube whistle, and the axial length of the first portion 11 of the open tube whistle 10 is greater than the axial length of the second portion 12 of the open tube whistle 10. The stepped profile of the inner diameter of the open tube whistle 10 acts as an acoustic impedance within the whistle 10, such that an audible/sound signal is only generated when a predetermined fluid flow rate is generated through the open tube whistle.

The second portion 12 of the open tube whistle 10 is adjacent to the second end 10b of the open tube whistle which forms the additional external opening of the pMDI 1, and the first portion 11 of the open tube whistle 10 is adjacent to the first end 10a of the open tube whistle 10 (when the open tube whistle is inserted into the pMDI 1).

To use the pMDI, a user places the mouthpiece 3 into their mouth and inhales. Air is drawn through the open tube whistle 10 via the additional external opening of the pMDI defined by the second end 10b of the open tube whistle 10. When the air flow through the open tube whistle 10 is at a predetermined air flow rate (which may be between 20 and 90 L/min), the stepped profile of the inner diameter of the open tube whistle 10 generates an audible/sound signal to indicate that the user is inhaling correctly. Therefore, a user is provided with instant feedback on their inhalation technique.

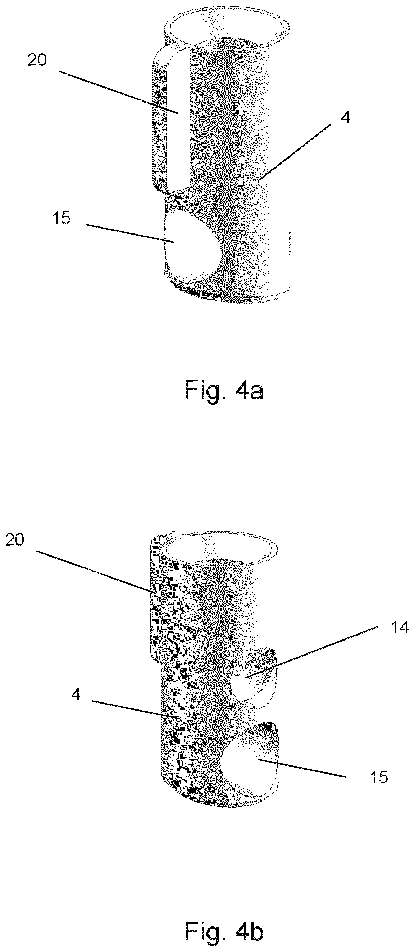

Figure 4a and 4b are different perspective views of the stem block 4 of the pMDI 1. The stem block 4 comprises a drug delivery output aperture 14. When the stem block is positioned in the main body 2, the drug delivery output aperture 14 is in fluid communication with the mouthpiece 3 such that the drug canister 5 can deliver a dose of drug through the drug delivery output aperture 14 into the mouthpiece 3 for inhalation by the user. Specifically, when a user actuates the drug canister 5 (typically by depressing the canister 5 downwards into the upright main body 2), an interaction between the canister 5 and the stem block 4 causes a metered dose of liquid drug to be ejected from the drug delivery output aperture 14 into the mouth of the user via the mouthpiece 3, along with a propellant gas. In this way, the liquid drug is aerosolised for inhalation by the user.

The stem block 4 has a passage 15 extending therethrough for receiving the open tube whistle 10. The passage 15 is spaced from the drug delivery output aperture 14. The stem block 4 also comprises a locating protrusion 20 for correctly positioning the stem block 4 in the main body 2.

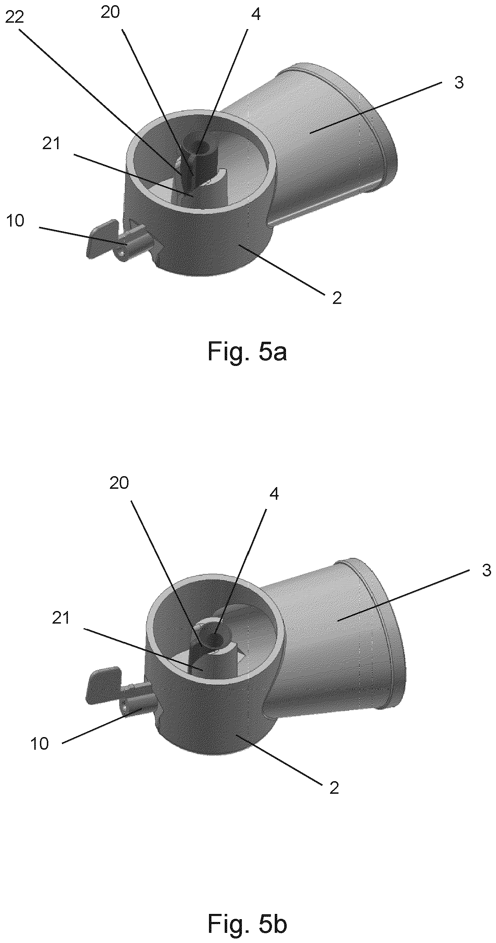

Specifically, as shown in Figure 5a, the stem block locating protrusion 20 helps to correctly position the stem block 4 in a stem block receiving portion 21 of the main body 2. The stem block receiving portion 21 has a corresponding locating recess 22 which interlocks with the stem block locating protrusion 20 when the stem block 4 is correctly positioned in the stem block receiving portion 21. Accordingly, the stem block 4 can be easily and accurately assembled in the main body 2. The stem block 4 can be pushed into and retained in the stem block receiving portion 21 by a push-fit fitting, as shown in Figure 5b.

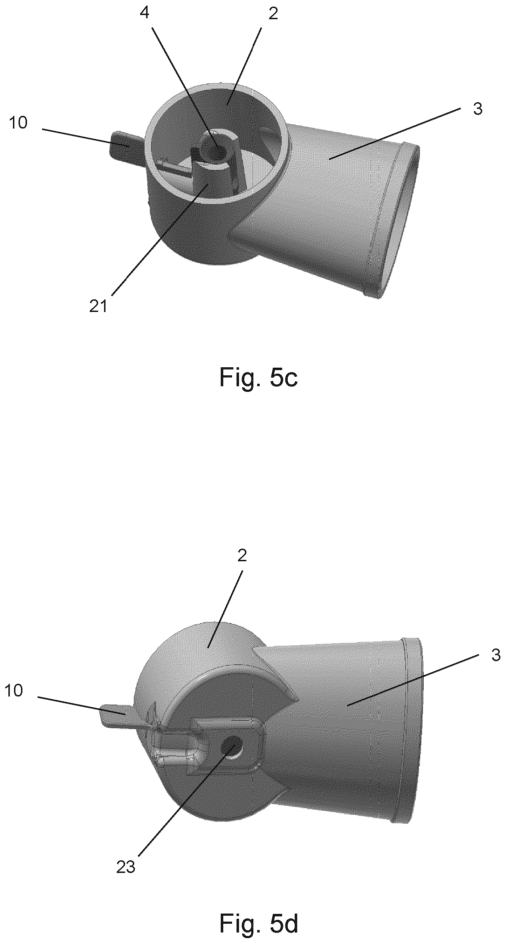

In Figure 5a and Figure 5b, the open tube whistle 10 is in a first position in which only the first end 10a of the open tube whistle is positioned within the pMDI 1. As shown in Figure 5c, after the stem block 4 has been pushed into the stem block receiving portion 21, the open tube whistle 10 is pushed through the passage 15 of the stem block 4, and into a second position in which the open tube whistle 10 extends through the stem block 4, and the first end 10a of the open tube whistle 4 is in fluid communication with the mouthpiece 3.

Accordingly, the open tube whistle 10 acts as a locking mechanism to lock the stem block 4 to the upright main body 2, by extending all the way through the stem block 4. The open tube whistle 10 is therefore incorporated as a sliding locking component to engage and disengage the removable stem block 4 from the main body 2.

As shown in Figure 5d, to remove the stem block 4 from the main body 2, the open tube whistle 10 is moved from the second position in which the open tube whistle 10 locks the stem block 4 into the stem block receiving portion 21, back into the first position in which the open tube whistle 10 does not extend through the stem block 4. The user may push the stem block out of the stem block receiving portion 21 via an opening 23 in an underside of the main body 2.

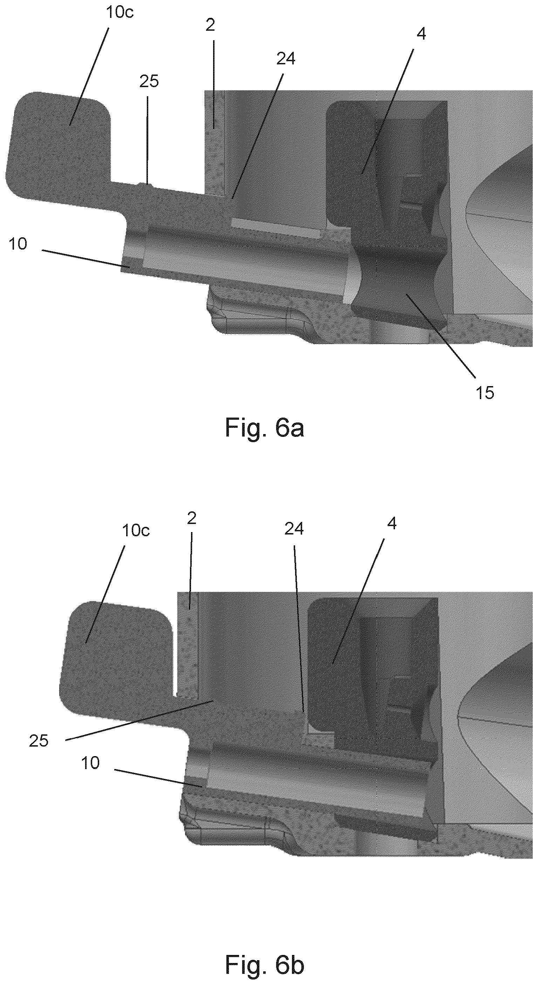

Figure 6a shows the open tube whistle 10 in the first position, and Figure 6b shows the open tube whistle 10 in the second position. As shown in Figure 6a, in the first position, the open tube whistle 10 does not extend through the passage 15 in the stem block 4. However, the first end 10a of the open tube whistle 10 is retained in the main body by a first projection 24. The first projection 24 of the open tube whistle 10 prevents the open tube whistle 10 from being completely removed from the main body 2, and therefore prevents the open tube whistle 10 from getting lost.

As shown in Figure 6b, in the second position, the open tube whistle 10 extends through the passage 15 in the stem block 4 to lock the stem block 4 to the main body 2. The stem block 4 is biased into the second position by a second projection 25 on the open tube whistle 10 with a snap-fit fitting. Specifically, the second projection 25 holds the open tube whistle 10 in the second position.

In alternative embodiments, the open tube whistle 10 may be biased into the second position by a spring loaded mechanism.

The handle tab 10c is used by the user to slide the open tube whistle 10 between the first position and the second position.

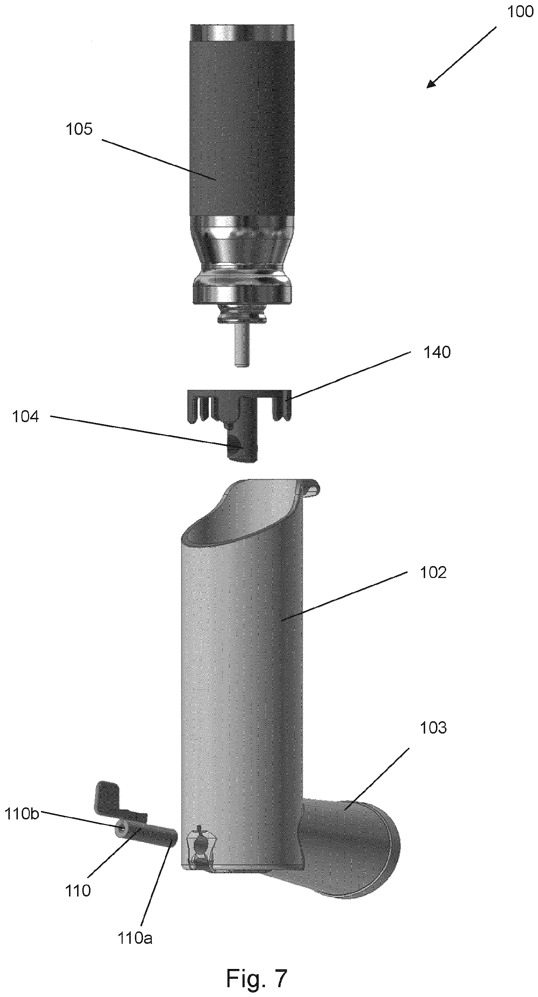

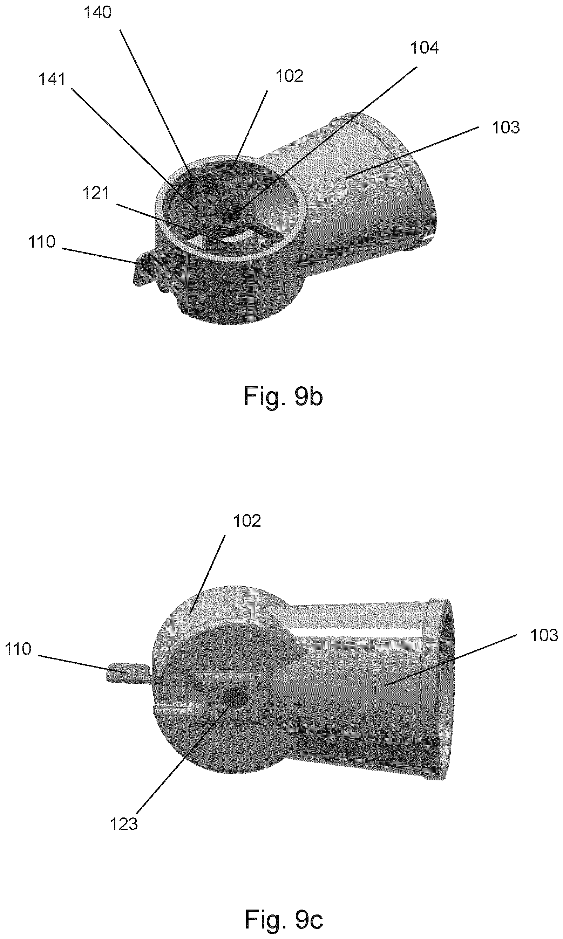

Figure 7 shows a pMDI 100 which is very similar to the pMDI 1 shown in Figure 1. Like reference numerals are used to indicate corresponding features. pMDI 100 is the same as pMDI 1 shown in Figure 1, except for that the stem block 104 has a stem block guider 140 for guiding the stem block 104 into the stem block receiving portion 121 (see e.g. Figure 9b) in the main body 102.

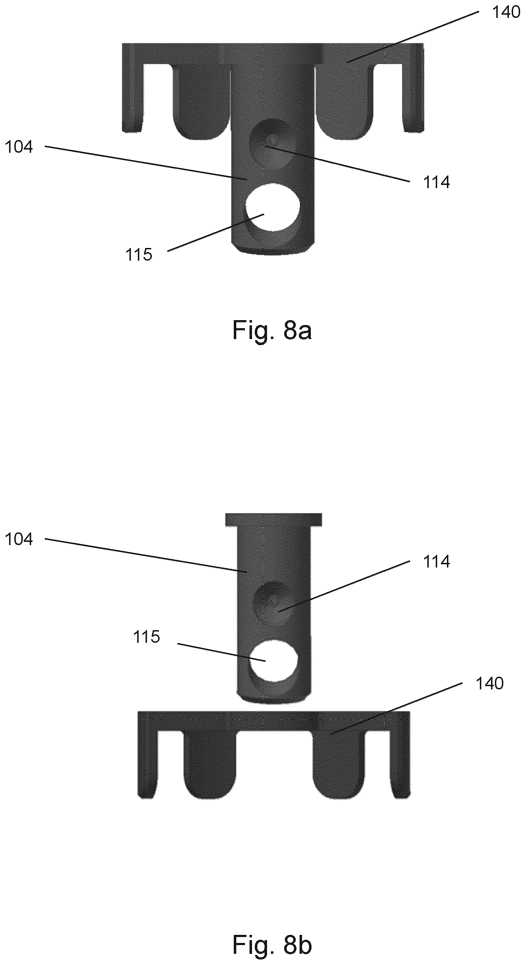

In Figure 8a, the stem block guider 140 is formed integrally with the stem block 104 as a single component. In Figure 8b, the stem block guider 140 is formed as a separate component from the stem block 104. Figure 8b is an exploded view of the stem block 104 and the stem block guider 140. In use, the stem block guider 140 is attached to the stem block 104.

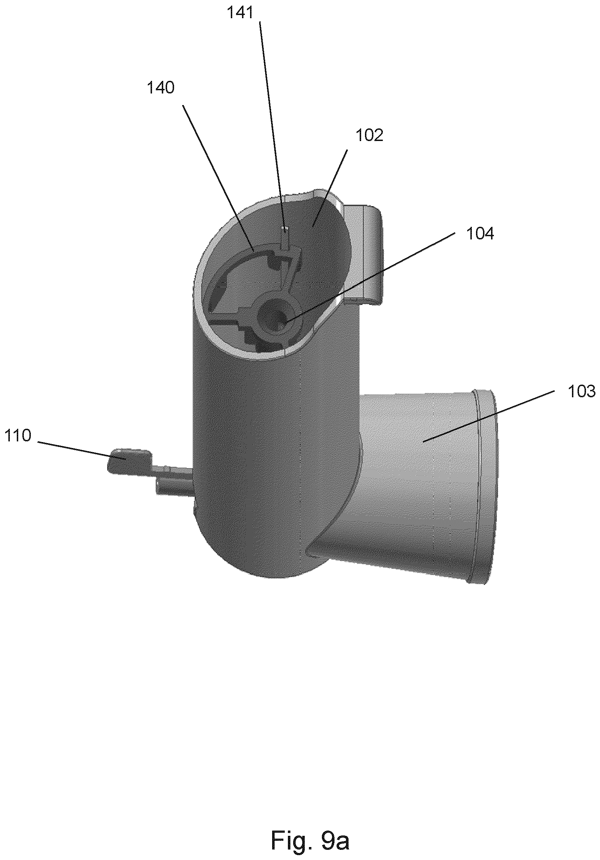

Figures 9a-c illustrate how the stem block guider 140 longitudinally slides along longitudinally extending ribs 141 in the main body to guide the stem block 104 into the stem block receiving portion 121 in the correct orientation. In Figure 9a, the open tube whistle 110 is in the first position, as previously described with reference to Figure 5a and 5b. When the stem block 104 is positioned in the stem block receiving portion 121, the open tube whistle 110 can be moved into the second position such that the open tube whistle extends through the passage in the stem block 104 (see e.g. Figure 9b). In this way, the open tube whistle 110 locks the stem block 104 to the main body 102.

Similarly to Figure 5d, and as shown in Figure 9c, to remove the stem block 104 from the main body 102, the open tube whistle 110 is moved from the second position in which the open tube whistle 110 locks the stem block 104 into the stem block receiving portion 121, back into the first position in which the open tube whistle 110 does not extend through the stem block 104. Then, a user may push the stem block 104 out of the stem block receiving portion 121 via an opening 123 in an underside of the main body 102.

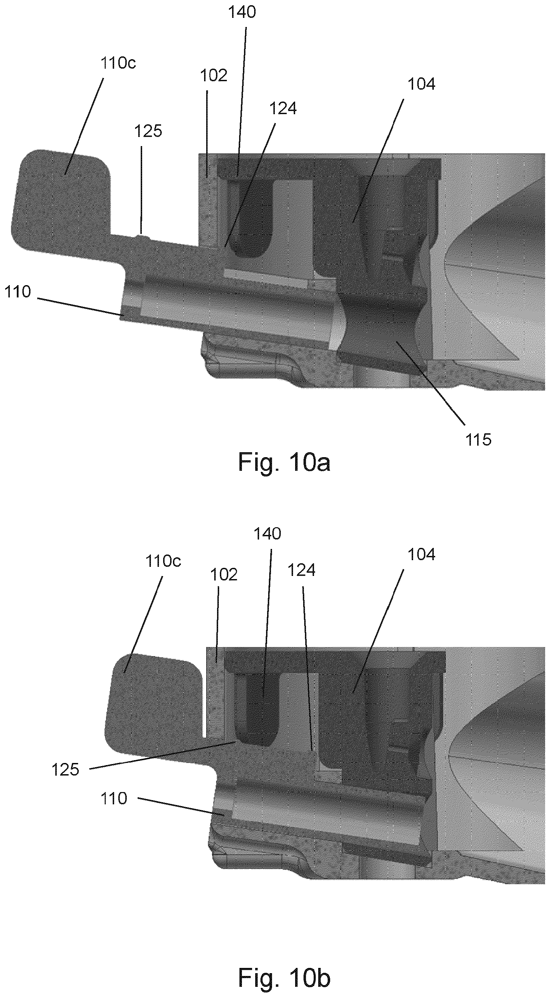

The longitudinally extending ribs 141 in the main body 102 of pMDI 100 have multiple uses. Ribs 141 can be used to help locate and guide drug canister 105 (shown in Figure 7), and specifically a canister valve stem of the drug canister 105, into the stem block 104. Ribs 141 can also be used to add strength to the main body 102. Furthermore, ribs 141 can also be used to guide the stem block into a correct position/orientation for interlocking with the open tube whistle. Figures 10a and 10b correspond to Figures 6a and 6b respectively. Figure 10a shows the open tube whistle 110 of pMDI in the first position, and Figure 10b shows the open tube whistle 110 in the second position. As shown in Figure 10a, in the first position, the open tube whistle 110 does not extend through the passage 115 in the stem block 104. However, the first end 110a of the open tube whistle 110 is retained in the main body 102 by a first projection 124. The first projection 124 of the open tube whistle 110 prevents the open tube whistle 110 from being completely removed from the main body 2, and therefore prevents the open tube whistle 110 from getting lost.

As shown in Figure 10b, in the second position, the open tube whistle 110 extends through the passage 115 in the stem block 104 to lock the stem block 104 to the main body 102. The stem block 104 is biased into the second position by a second projection 125 on the open tube whistle 110 with a snap-fit fitting. Specifically, the second projection 125 holds the open tube whistle 110 in the second position.

In alternative embodiments, the open tube whistle 110 may be biased into the second position by a spring loaded mechanism.

The handle tab 110c is used by the user to slide the open tube whistle 110 between the first position and the second position.

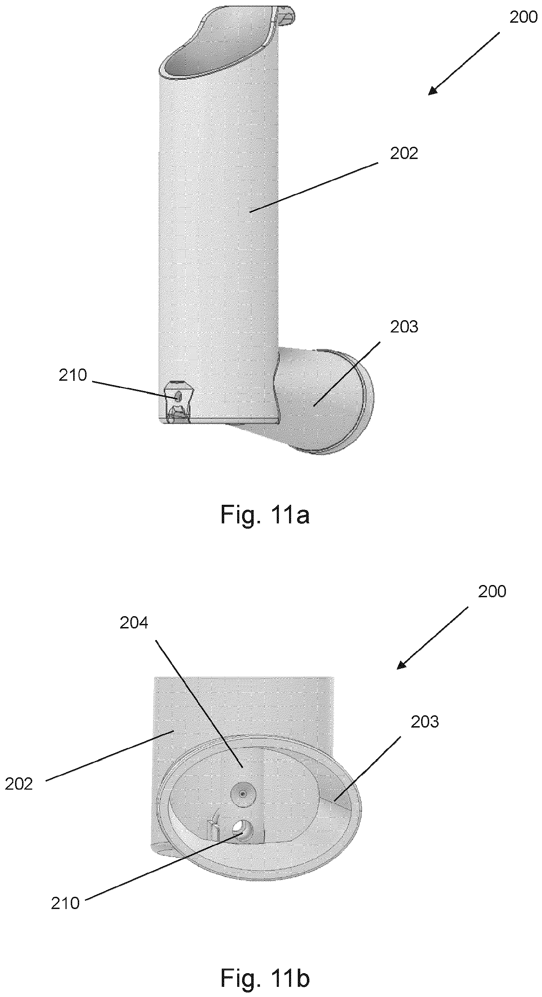

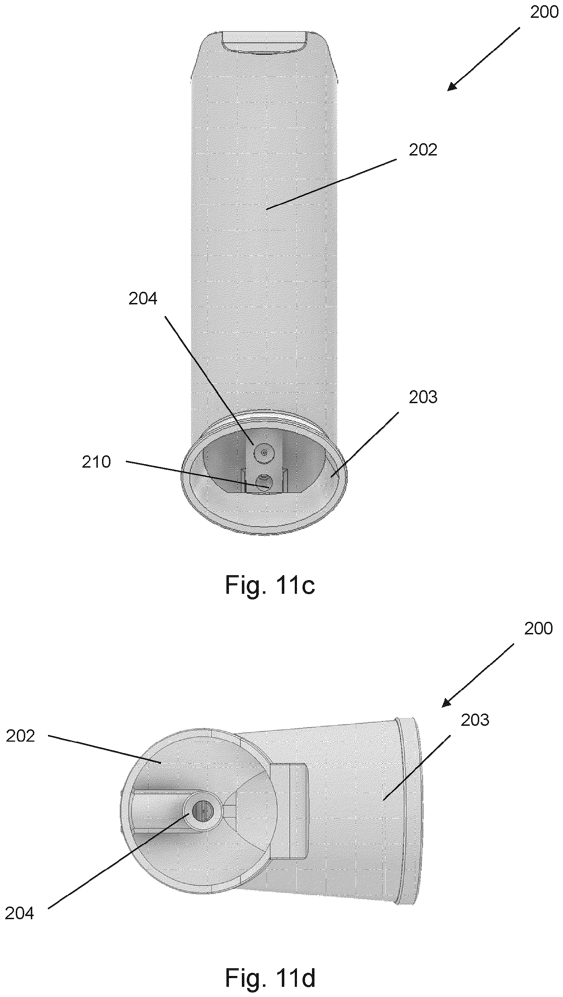

Figures 11a-d show different perspective views of a pMDI 200 according to an alternative embodiment. pMDI 200 is similar to pMDI 1 and pMDI 100 except for that the main body 202, mouthpiece 203, stem block 204 and open tube whistle 210 are integrally formed as a single component. Accordingly, although this embodiment is not as sustainable as pMDI 1 or pMDI 100 (because the uncontaminated parts such as the main body and the open tube whistle cannot be removed from the drug-contaminated parts, and reused), it does not require assembling and so is easy to use.

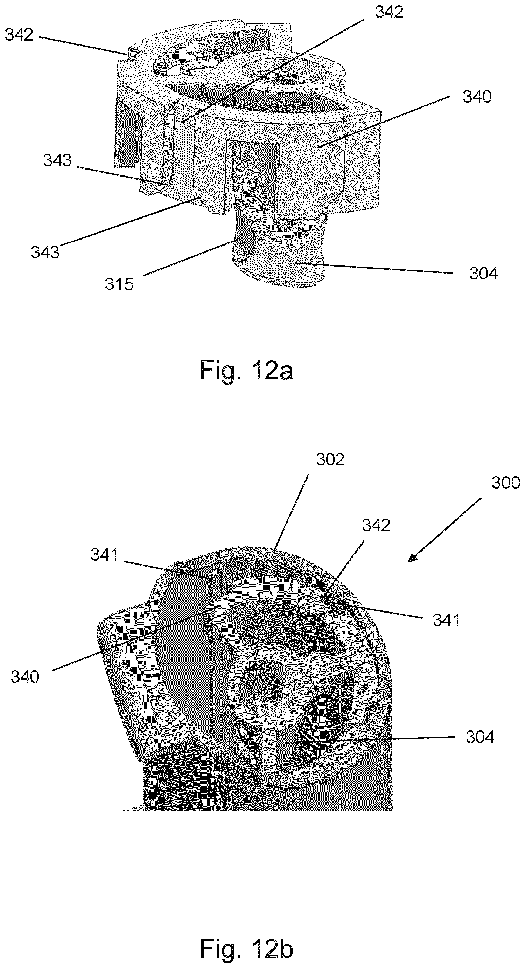

Figures 12a and 12b illustrate stem block guider 340, which is similar to stem block guider 140 as shown in Figures 8a-9c, and so like reference numerals are shown. Stem block guider 340 comprises a plurality of channels 342 which correspond to longitudinally extending ribs 341 in the main body 302 of pMDI 300 (which is similar to pMDI 100) such that stem block guider 340 can longitudinally slide along the longitudinally extending ribs 341 (see e.g. Figure 12b). The channels 342 may comprise chamfers 343, which help to correctly align the channels 342 with respective longitudinally extending ribs 341 when the stem block 304 and stem block guider 340 are introduced into the main body 302. This may help to correctly align the stem block 304 in the main body 302.

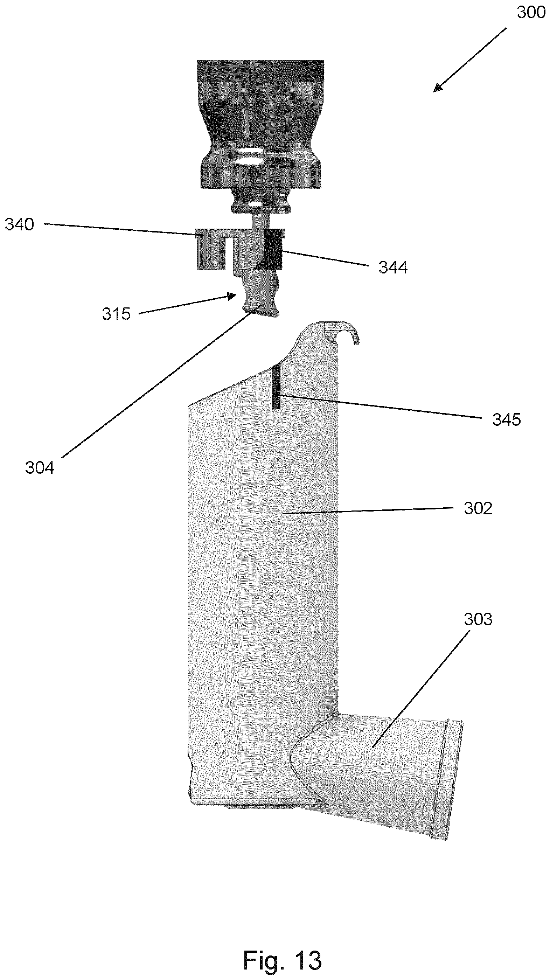

pMDI 300 is illustrated in Figure 13, and is similar to pMDI 100. Therefore, like reference numerals are shown. pMDI 300 differs from pMDI 100 in that stem block guider 340 comprises a location indicating feature 344. Main body 302 of pMDI 300 comprises a corresponding location indicating feature 345. The location indicating feature 345 on the main body 302 is located at an end of the main body 302 distal to mouthpiece 303, and is located on an external surface of the main body 302 (although in other embodiments, the location indicating feature 345 may be located on an internal surface of the main body).

The location indicating features 344, 345 may help a user to correctly align the stem block 304 in a correct orientation in the main body 302, and specifically in the stem block receiving portion of the main body 302. The correct orientation may be an orientation in which an open tube whistle (such as open tube whistle 10, 110 described above) may be inserted into the main body through passage 315 in the stem block 304. A user may align the location indicating features 344, 345 on the stem block guider 340 and main body 302 in order to position the stem block 304 in the main body 302 in the correct orientation.

In alternative embodiments, only one of the stem block guider and the main body may have a location indicating feature.

In Figure 13, the location indicating features 344, 345 on the stem block guider 340 and main body 302 comprise visual location indicating features. The visual location indicating features 344, 345 are colour coded (e.g. have the same colour) and are manufactured by injection moulding (e.g. two shot injection moulding). In alternative embodiments, the visual location indicating features 344, 345 may comprise stickers. In some embodiments, the location indicating features may comprise protrusions and corresponding cut-outs, or may be formed from portions of the stem block guider/main body having a different surface texture to the remaining portion of the stem block guider/main body, or may comprise the Braille tactile reading and writing system. For example, a majority of the stem block guider may have a smooth surface texture, and the location indicating features may have a rough surface texture.

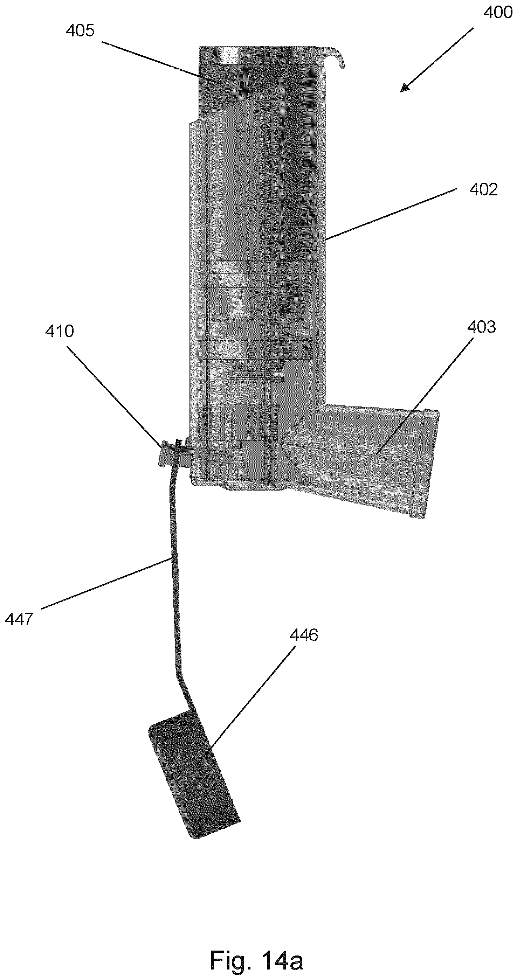

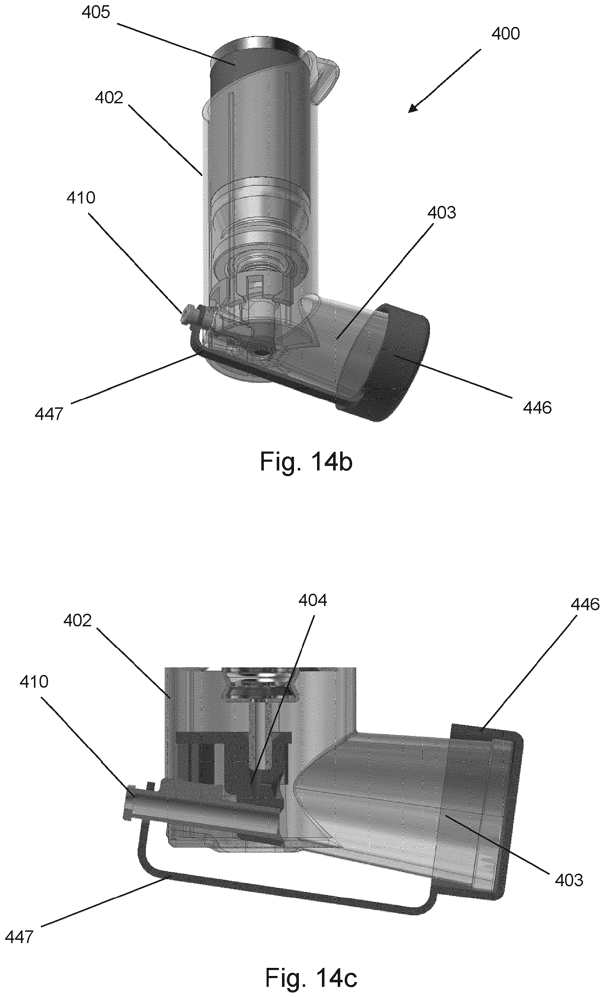

Figures 14a-14c illustrate a pMDI 400 which is similar to pMDIs 1, 100, 300 and so like reference numerals are shown. However, pMDI 400 additional comprises a mouthpiece cap 446 which is tethered to the open tube whistle 410. As shown in Figure 14c, mouthpiece cap 446 can cover the entire opening in the mouthpiece 403 to ensure the opening in the mouthpiece 403 remains clean when not in use. As the mouthpiece cap 446 is tethered to the open tube whistle 410, the mouthpiece cap 446 cannot be lost when removed from the mouthpiece 403. Furthermore, open tube whistle 410 cannot be lost from the main body 402 when the mouthpiece cap 446 is positioned over the mouthpiece 403.

Mouthpiece cap 446 is attached to open tube whistle 410 via tether 447, which is formed integrally with the mouthpiece cap 446. However, in other embodiments, the tether 447 and mouthpiece cap 446 may be formed as separate components. In order to attach the tether 447 to the open tube whistle 410, the length of the open tube whistle is extended compared to that shown in 6a and 6b, for example. Accordingly, open tube whistle 410 may generate an audible/sound signal with a lower frequency than the audible/sound signal generated by open tube whistle 10 shown in Figures 6a and 6b.

In Figures 14a-c, open tube whistle 410, stem block 404 and mouthpiece cap 446 are all manufactured as separate components. Main body 402 is also manufactured as a separate component. In this embodiment, and similarly to open tube whistle 10 in pMDI 1, open tube whistle 410 is reversibly receivable in the stem block 404, as described above with reference to Figures 6a and 6b.

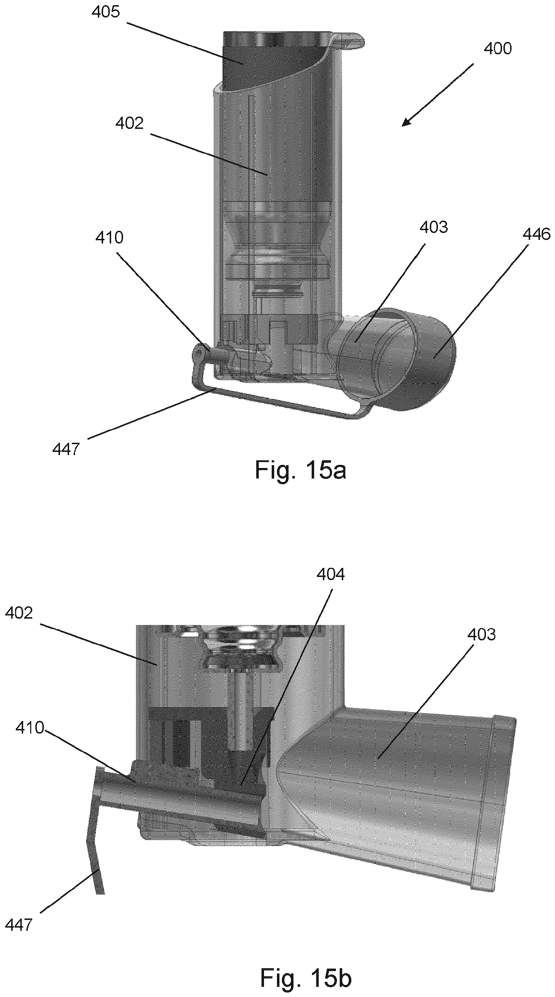

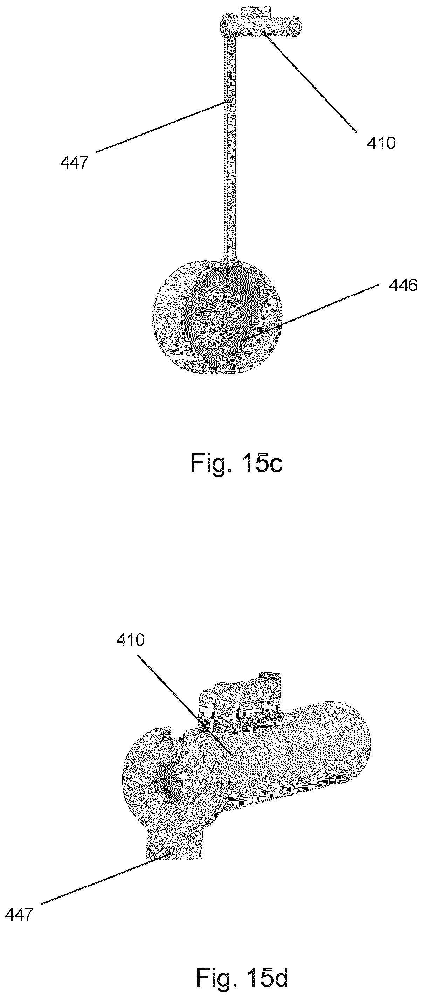

Figures 15a-d illustrate an alternative embodiment in which the mouthpiece cap 446 and the open tube whistle 410 (and tether 447) are integrally formed as a single component, but stem block 404 is formed as a separate component. Main body 402 is also manufactured as a separate component. In this embodiment, and similarly to in Figures 14a-14c, open tube whistle 410 is reversibly receivable in stem block 404. Figures 15c and 15d show the open tube whistle 410, tether 447 and open tube whistle 410 integrally formed as a single component.

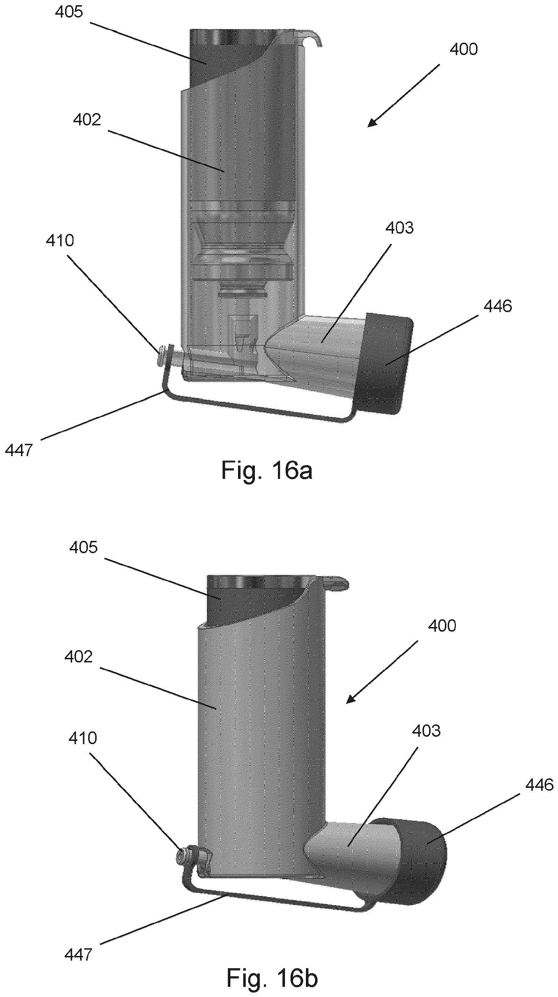

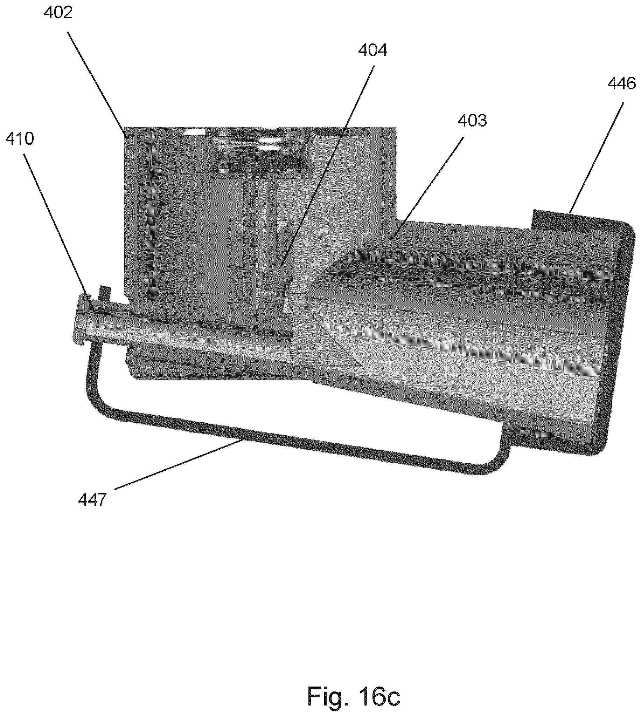

Figures 16a-c illustrate a further alternative embodiment in which main body 402, mouthpiece 403, stem block 404 and open tube whistle 410 are integrally formed as a single component (similarly to pMDI 200 as shown in Figures 11a-d). Mouthpiece cap 446 and tether 447 are integrally formed as a separate single component, and the mouthpiece cap 446 is attached to the open tube whistle 410 via tether 447.

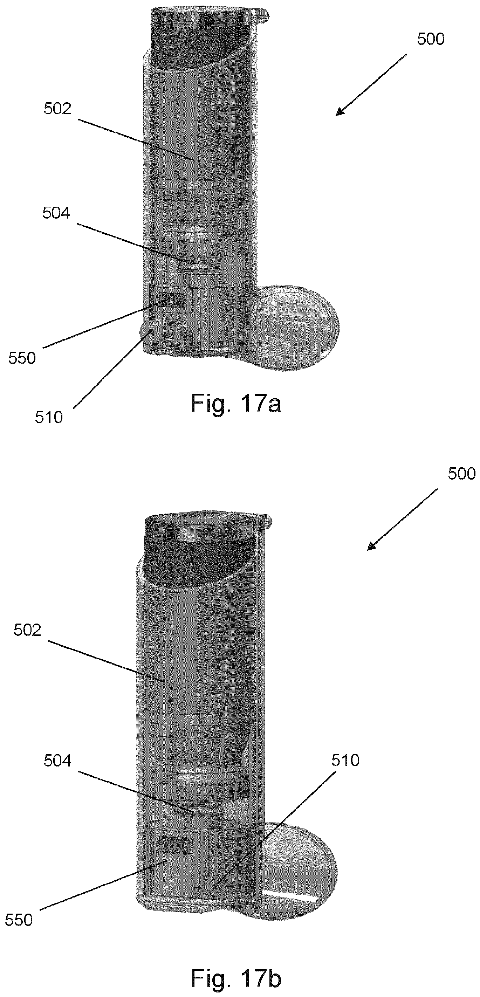

Figures 17a and 17b illustrate embodiments of the inhaler device in which a counter 550 is arranged within the main body 502 of pMDI 500.

In Figure 17a, the counter 550, open tube whistle 510, main body 502 and stem block 504 are formed as separate components. The open tube whistle 510 is slidably and reversibly receivable in the counter 510. The open tube whistle 510 extends through the counter 550 in order to lock the counter 550 to the main body 502.

In Figure 17b, the counter 550 and open tube whistle 510 are integrally formed as a single component.

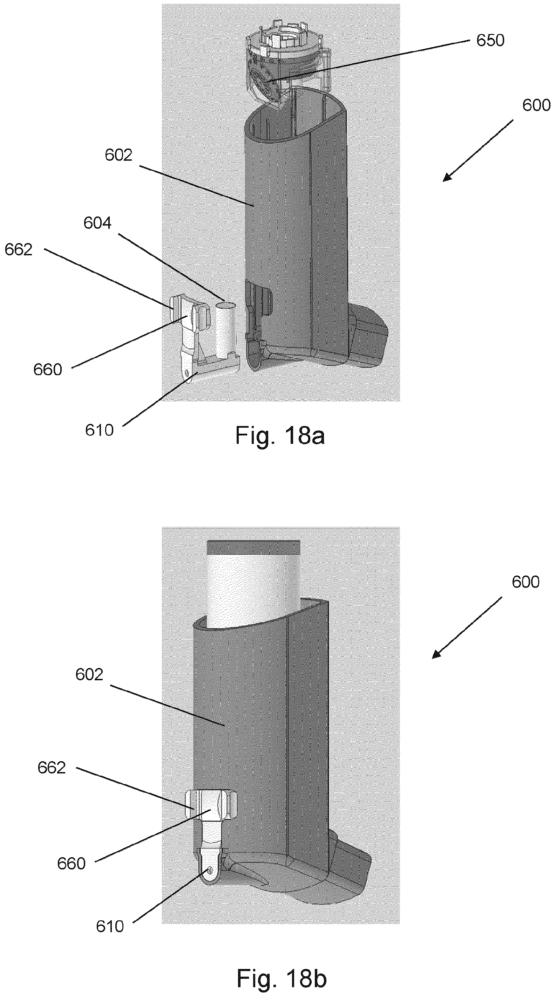

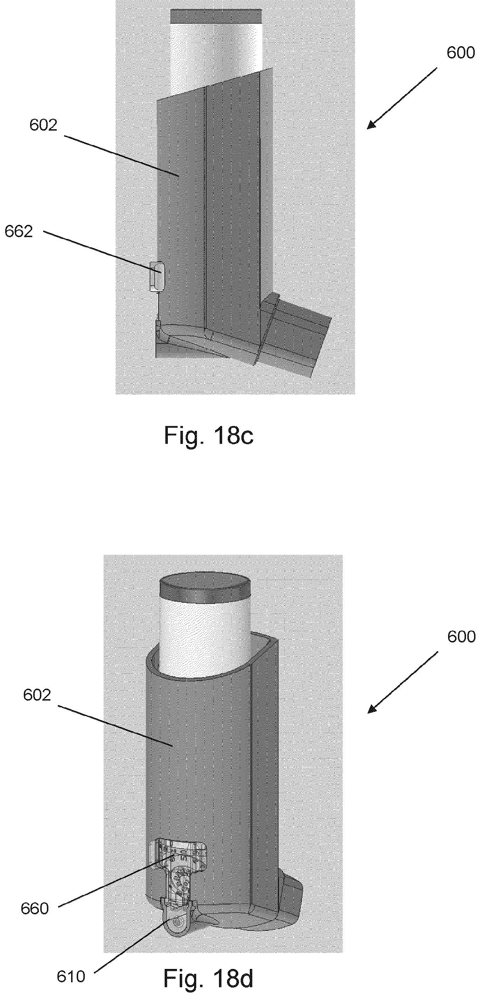

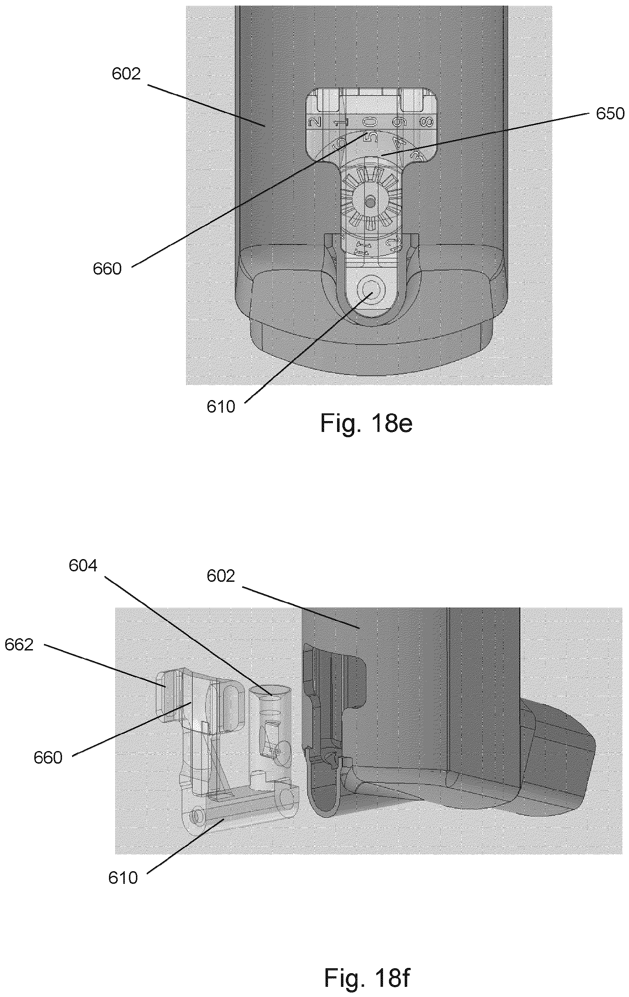

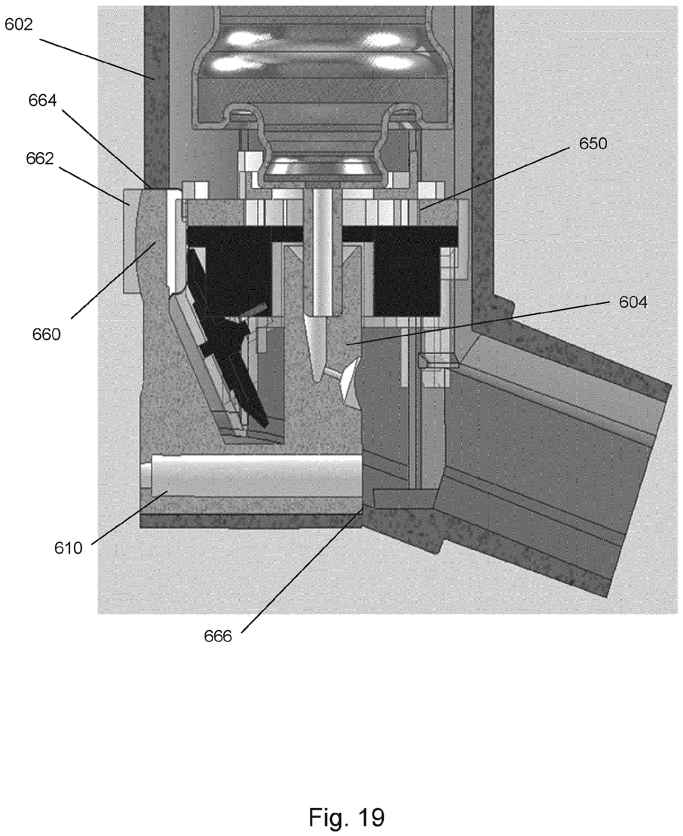

Figures 18a-f and Figure 19 illustrate further embodiments of an inhaler device having a counter 650 arranged within the main body 602 of pMDI 600. The inhaler device further comprises a magnifying lens 660 arranged to enhance the visibility of the counter 650 arranged within the main body 602. Specifically, the magnifying lens 660 magnifies the number of actuations of the pMDI displayed on the counter 650, as best shown in Figure 18e.

As best shown in Figures 18a and 18f, the magnifying lens 660, open tube whistle 610 and stem block 604 are integrally formed as a single component that can be slidably received in the main body 602 by a snap-fit connection. Specifically, as best shown in Figure 19, bumps and/or tapers 664 (i.e. angled faces) may be formed on one or more of the main body 602, magnifying lens 660, stem block 604 and open tube whistle 610 to provide an interference or snap-fit connection when the single component formed from the magnifying lens 660, open tube whistle 610 and stem block 604 is slidably received in the main body 602. For example, bumps may be located on the magnifying lens 660 to provide a snap-fit (e.g. clip) connection with the main body 602. The main body 602 may be chamfered to guide the single component formed from the magnifying lens 660, open tube whistle 610 and stem block 604 thereinto. The single component formed from the magnifying lens 660, open tube whistle 610 and stem block 604 and/or the main body 602 may have angled or tapered faces to provide an interference fit when the single component is slidably received in the main body 602.

When the single component formed from the magnifying lens 660, open tube whistle 610 and stem block 604 is received in the main body 602, the single component may be secured (e.g. locked) in three dimensions within the main body 602. The single component and/or the main body 602 may comprise one or more securing elements for securing the single component within the main body 602 in three dimensions. For example, the main body 602 may comprise one or more securing protrusions and the single component may comprise one or more securing grooves (or vice versa). The securing protrusions and the securing grooves may interlock when the single component is received in the main body 602 to prevent vertical displacement of the single component (e.g. displacement in a direction parallel to a central longitudinal axis of the main body 602) in the main body 602.

A pair of assembly tabs 662 are formed either side of the magnifying lens 660 to aid the insertion and removal of the single component from the main body 602. As best shown in Figure 19, a stop face 666 of the main body 602 ensures that the open tube whistle 610 is correctly positioned inside the main body 602.

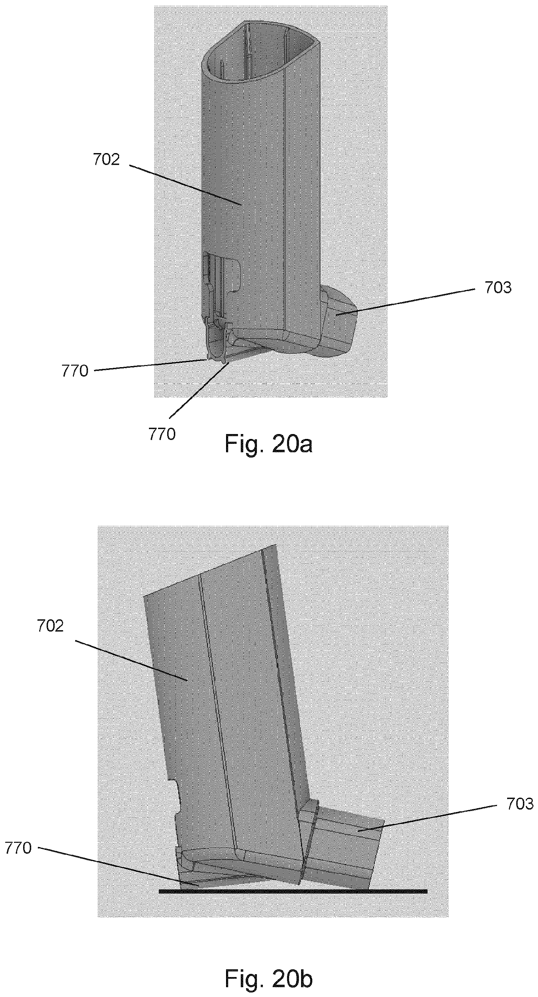

Figures 20a and 20b illustrate an integrally formed main body 702 and mouthpiece 703 for an inhaler device such as the pMDIs described above. A pair of balance protrusions 770 extend from the lower end of the main body 702. The balance protrusions 770 provide points of contact with a flat surface when the bottom surface of the inhaler device is resting on the flat surface. Specifically, as shown in Figure 20b, the balance protrusions 770 and the mouthpiece 703 provide points of contact with the flat surface to improve stability of the inhaler device when the device stands upright on a flat surface.

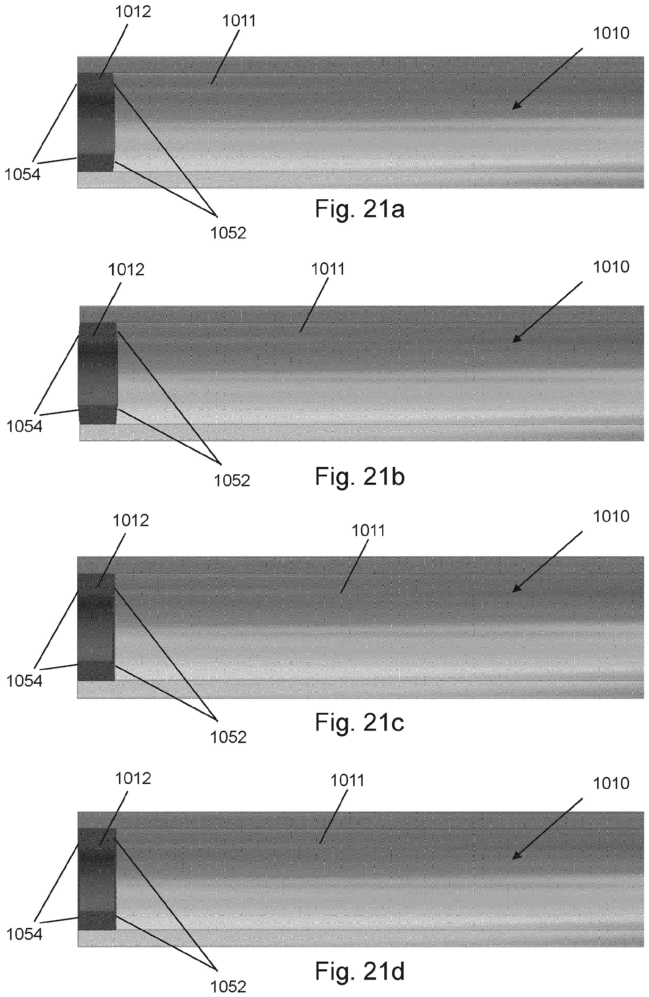

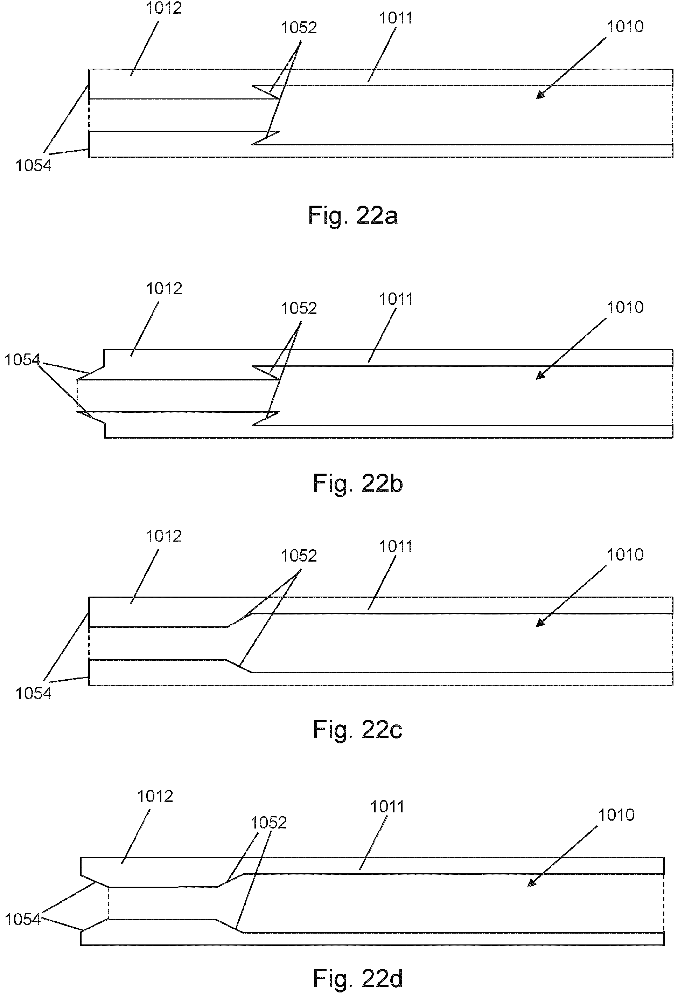

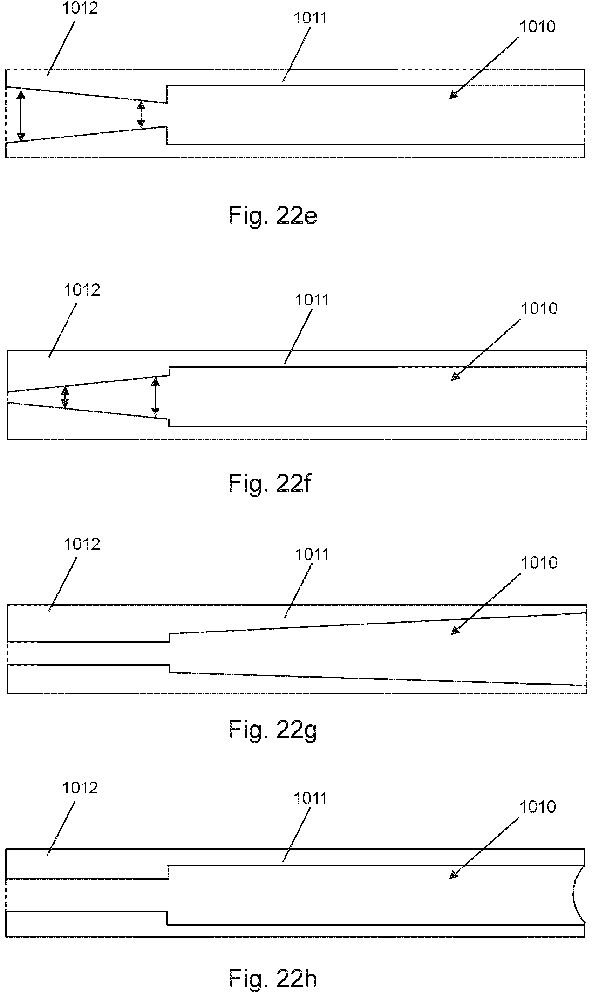

Figures 21a-21h illustrate various possible cross-sectional profiles of open tube whistle 1010 which may be incorporated into any of the pMDIs described above, or any of the oral mask assemblies described below. Figures 22a-22h corresponds to Figures 21a-21h respectively, with exaggerated cross-sectional profiles.

Figures 21a-21d illustrate open tube whistles 1010 having internal step faces 1052 between the first portion 1011 and second portion 1012 of the open tube whistles 1010, and external step faces 1054, which correspond to the end face of the open tube whistle 1010 adjacent to the second portion 1012 of the open tube whistle. As described herein, flat step faces are understood as being perpendicular to the axial length of the open tube whistle, whereas angled step faces are understood as being not perpendicular to the axial length of the open tube whistle.

The open tube whistle 1010 of Figure 21a has angled internal step faces 1052, but flat external step faces 1054. The angled internal step faces 1052 are angled outwardly such that the second portion 1012 of the open tube whistle 1010 has a greater axial length at a region closer to a central longitudinal axis of the open tube whistle 1010 than at a region further from a central longitudinal axis of the open tube whistle 1010.

The open tube whistle 1010 of Figure 21b has both angled internal step faces 1052 and angled external step faces 1054. The angled internal and external step faces 1054 are both angled outwardly such that the second portion 1012 of the open tube whistle 1010 has a greater axial length at a region closer to a central longitudinal axis of the open tube whistle 1010 than at a region further from a central longitudinal axis of the open tube whistle 1010.

The open tube whistle 1010 of Figure 21c has angled internal step faces 1052, and flat external step faces 1054. The angled internal step faces 1052 are angled inwardly such that the second portion 1012 of the open tube whistle 1010 has a smaller axial length at a region closer to a central longitudinal axis of the open tube whistle 1010 than at a region further from a central longitudinal axis of the open tube whistle 1010.

The open tube whistle 1010 of Figure 21d has both angled internal step faces 1052 and angled external step faces 1054. The angled internal and external step faces 1054 are both angled inwardly such that the second portion 1012 of the open tube whistle 1010 has a smaller axial length at a region closer to a central longitudinal axis of the open tube whistle 1010 than at a region further from a central longitudinal axis of the open tube whistle 1010.

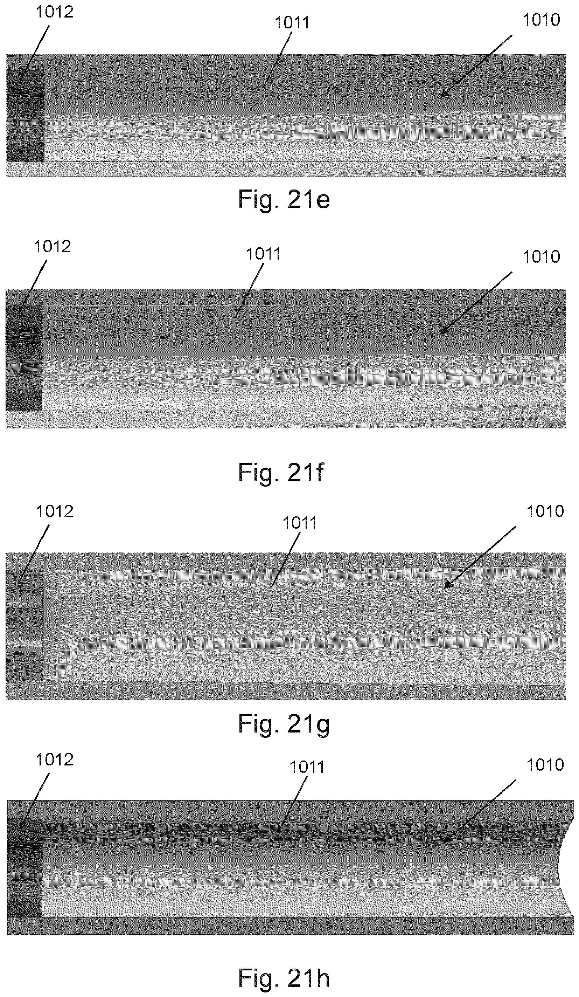

Figures 21e and 21f illustrate open tube whistles 1010, wherein the inner diameter of the second portion 1012 of the open tube whistle 1010 tapers along its axial length. In Figure 21e, the inner diameter of the second portion 1012 of the open tube whistle 1010 tapers towards the first portion 1011 of the open tube whistle. In Figure 21f, the inner diameter of the open tube whistle 1010 tapers away from the first portion 1011 of the open tube whistle 1010.

Figure 21g illustrates an open tube whistle 1010, wherein the inner diameter of the first portion 1011 of the open tube whistle 1010 tapers along its axial length. Specifically, the inner diameter of the first portion 1011 of the open tube whistle 1010 tapers towards the second portion 1012 of the open tube whistle 1010. Although not shown, alternative open tube whistles may be formed such that an inner diameter of the first portion of the open tube whistle tapers away from the second portion of the open tube whistle.

Figure 21h illustrates an open tube whistle 1010, wherein the first end of the open tube whistle 1010 (for fluid communication with the mouthpiece), and adjacent the first portion 1011 of the open tube whistle 1010 is non-planar. In this example, the first end of the open tube whistle 1010 is concave.

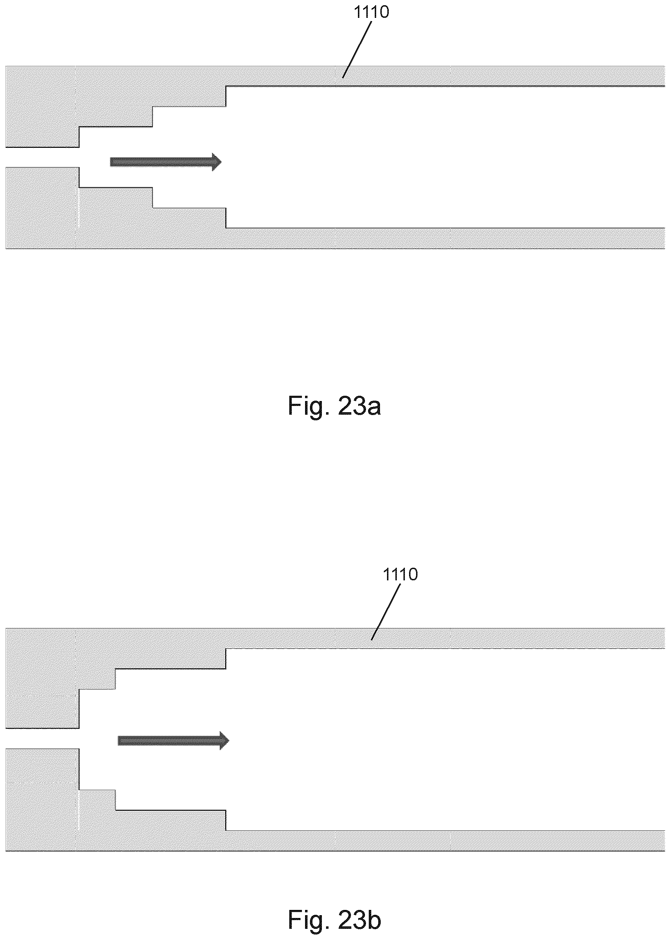

Figures 23a and 23b illustrate cross-sectional profiles of open tube whistle 1110 which may be incorporated into any of the pMDIs described above, or any of the oral mask assemblies described below. The stepped profile of open tube whistle 1110 comprises a plurality of steps. In Figure 23a, each step has a similar height and width (e.g. relative to neighbouring steps), whereas in Figure 23b, the steps have different heights and widths (e.g. relative to neighbouring steps). The height and width of the steps may be chosen to generate sound with a specific (e.g. preferred) frequency. The arrows in Figures 23a and 23b indicate the direction of fluid (e.g. air) flow through the open tube whistle 1110, in use.

The audible/sound signal generated by open tube whistle 10, 110, 210, 310, 410, 510, 610 may be detectable by a sound receiver on a mobile device, such as a mobile phone. The mobile device may be configured to monitor, record and/or analyse the fluid flow rate through the open tube whistle and/or breathing patterns based on the frequencies/harmonics of the audible/sound signal detected by the sound receiver.

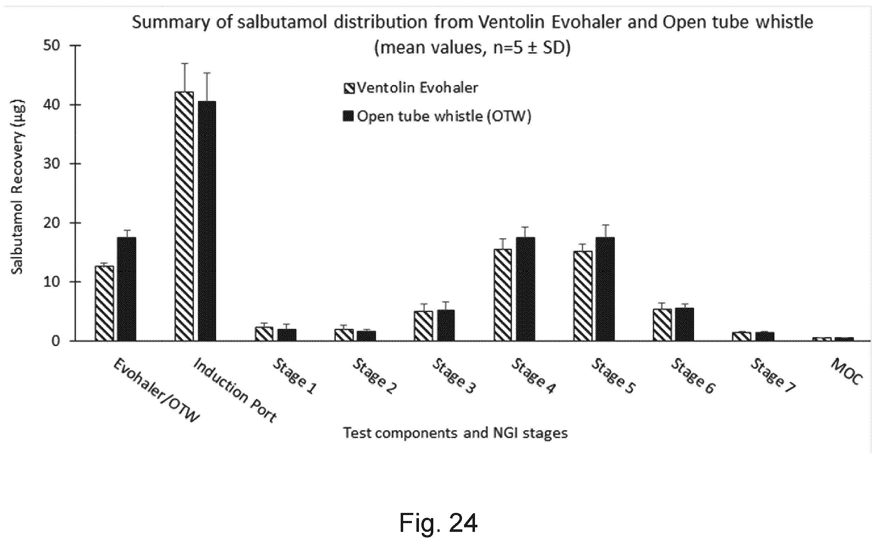

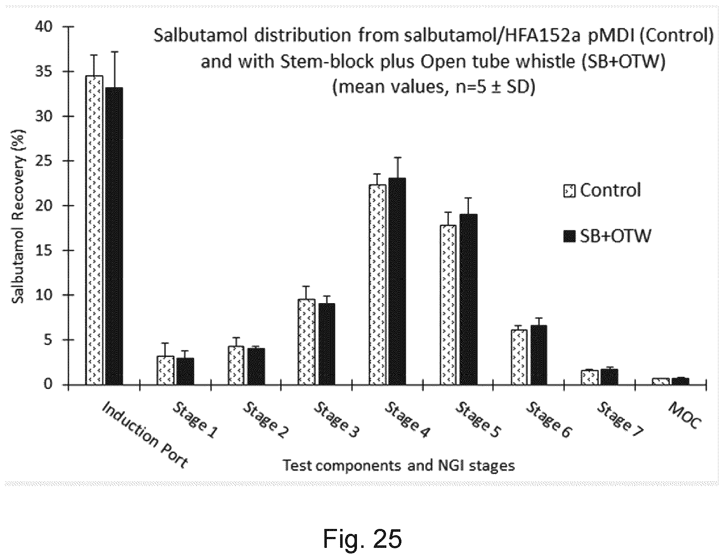

Figure 24 is a graph showing a comparison of the performance of an inhaler device comprising an open tube whistle (labelled as "OTW"), with an inhaler device without an open tube whistle (labelled as "Ventolin Inhaler"), based on experimental data from experiments conducted by the inventors. Figure 25 is a graph showing a comparison of the performance of a pMDI comprising a salbutamol/HFA152a formulation, and an integral stem block and open tube whistle (labelled as "SB+OTW") with a standard pMDI comprising a salbutamol/HFA152a formulation (labelled as "CONTROL"), based on experimental data from experiments conducted by the inventors. As shown in Figures 24 and 25, performance of the inhaler device is not unduly compromised by the presence of an open tube whistle. Therefore, the presence of the open tube whistle, which provides feedback to the user that they have the correct inhalation technique, does not unduly affect the performance of the inhaler device to deliver an active ingredient/drug to a user.

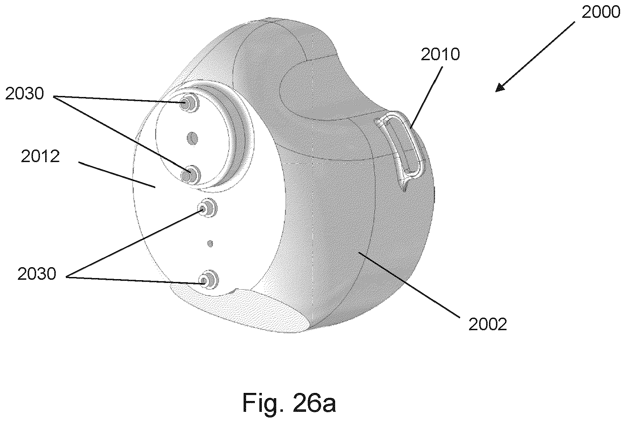

Figures 26a-26d show different views of an oral mask assembly 2000. The oral mask assembly 2000 comprises a breathing mask 2002 which is configured to be worn by a user so that the breathing mask 2002 covers the mouth and nose of the user. The oral mask assembly 2000 comprises strap loop buckles 2010 for attaching to a strap for fixing the breathing mask 2002 to the user's face. A main body 2012 of the breathing mask 2002 is injection moulded from a polymer material such as PVC, ABS or PP, and a rim of the main body 2012 is lip-shaped and comprises rubber, a plastic/polymer, silicone and/or foam (e.g. Polyvinyl Chloride, PVC), for providing a tight and comfortable seal with the user's face.

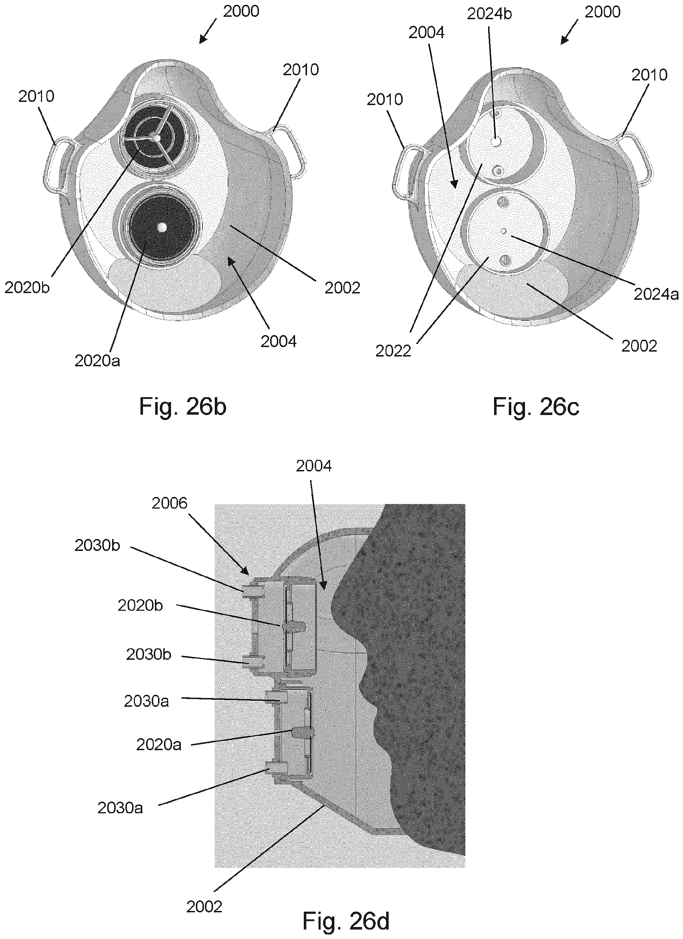

As shown in Figures 26b and 26d, the oral mask assembly 2000 comprises two one-way valves 2020a, 2020b, one for user inhalation and the other for user exhalation. As shown in Figure 26b and 26c, the one-way valves 2020a, 2020b are attachable to the main body 2012 of the breathing mask 2002 in a pair of valve receiving recesses 2022 by way of a snap-fit connection. The valve receiving recesses 2022 are formed on an internal side 2004 of the main body 2012 of the breathing mask 2002.

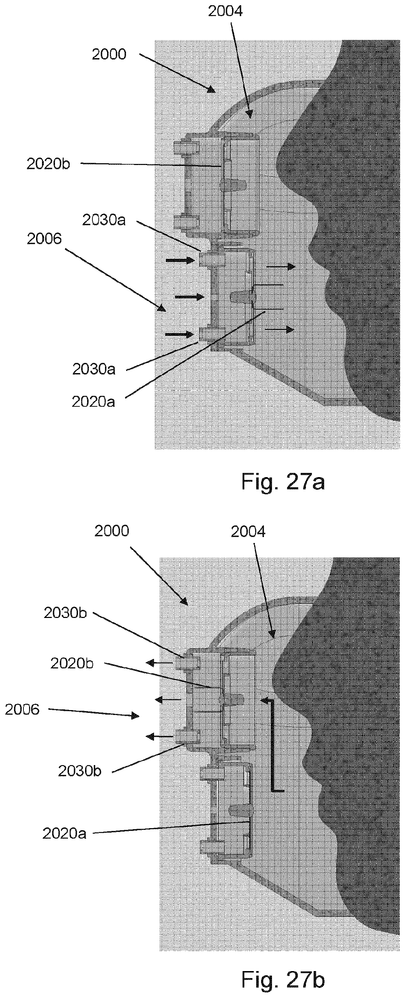

Each valve receiving recess 2022 comprises a bypass channel 2024a, 2024b, as best shown in Figure 26c. When the one-way valves 2020a, 2020b are received in the valve receiving recesses 2022, each of the one-way valves 2020a, 2020b is in fluid communication with a respective bypass channel 2024a, 2024b. As shown in Figure 26c, 27a and 27b, the inner diameter of the bypass channels 2024a, 2024b are different. In particular, the inner diameter of the bypass channel 2024b in fluid communication with the one-way valve 2020b for user exhalation is greater than the inner diameter of the bypass channel 2024a in fluid communication with the one-way valve 202a for user inhalation 2020a.

Although not shown in the figures, the one-way valves 2020a, 2020b may comprise a filter, such as an antimicrobial medical grade filter. A filter may be attachable to each of the one-way valves, and may be configured to be fitted to the respective one-way valve adjacent to the internal side 2004 and/or the external side 2006 of the breathing mask 2002.

The oral mask assembly 2000 comprises a plurality, in this case four, open tube whistles 2030a, 2030b. As described above in relation to previous examples, the open tube whistles 2030a, 2030b have a stepped profile and each open tube whistle 2030a, 2030b generates an audible/sound signal at a predetermined fluid flow rate through the open tube whistle. One end of each open tube whistle 2030a, 2030b is in fluid communication with an external side 2006 of the breathing mask 2002, and the other end of each open tube whistle 2030a, 2030b is in fluid communication with the internal side 2004 of the breathing mask 2002.

In Figure 26d, two open tube whistles 2030a, 2030b are in fluid communication with a respective one-way valve 2020a, 2020b (e.g. two open tube whistles 2030a are in fluid communication with the one-way valve 2020a for fluid inhalation, and two open tube whistles 2030b are in fluid communication with the one-way valve 2020b for fluid exhalation). The open tube whistles 2030a, 2030b are integrally moulded with the main body 2012 of the breathing mask 2002. Alternatively, one or more of the open tube whistles 2030a, 2030b may be attachable to (e.g. slidably received in) the main body 2012. The open tube whistles 2030a, 2030b may be formed in one or more additional components, wherein the one or more additional components are attachable to (e.g. slidably received in) the main body 2012.

This would allow for the open tube whistles 2030a, 2030b to be easily replaced, for example with open tube whistles of different tube geometries to thereby generate an audible/sound signal at a different fluid flow rate therethrough. Resistance of fluid flow between the internal and external sides of the breathing mask may also be adjusted by replacing open tube whistles 2030a, 2030b with open tube whistles of different tube geometries.

Figures 27a and 27b illustrate air flow through the oral mask assembly 2000 during user inhalation and user exhalation, respectively. In Figure 27a, arrows indicate the air flow path through a first one-way valve 2020a which opens to permit fluid flow from the external side 2006 to the internal side 2004 of the breathing mask 2002 as the user inhales (but prohibits air flow in the other direction by closing when the user exhales). During user inhalation, air also flows through two open tube whistles 2030a, which each generate an audible/sound signal at a specific rate of air flow during user inhalation. The stepped profiles of the two open tube whistles 2030a for user inhalation may have the same/similar or different dimensions or geometries (as described above) to each other, such that the two open tube whistles 2030a generate an audible/sound signal at different predetermined fluid flow rates. An inner diameter of a first portion of the open tube whistles 2030a for user inhalation is greater than an inner diameter of a second portion of the open tube whistles 2030a for user inhalation. The first portion having the greater diameter of each open tube whistle 2030a for user inhalation is provided in fluid communication and adjacent to the internal side 2004 of the breathing mask 2002. The second portion having the smaller inner diameter of each open tube whistle 2030a for user inhalation is provided in fluid communication and adjacent to the external side 2006 of the breathing mask 2002.

In Figure 27b, the arrows indicate the air flow path through a second one-way valve 2020b which opens to permit fluid flow from the internal side 2004 to the external side 2006 of the breathing mask 2002 as the user exhales (but prohibits air flow in the other direction by closing when the user inhales). During user exhalation, air also flows through two open tube whistles 2030b, which each generate an audible/sound signal at a specific rate of air flow during user exhalation. The stepped profiles of the two open tube whistles 2030b for user exhalation may have different dimensions or geometries (as described above), such that the two open tube whistles 2030b generate an audible/sound signal at different predetermined fluid flow rates during user exhalation. An inner diameter of a first portion of the open tube whistles 2030b for user exhalation is greater than an inner diameter of a second portion of the open tube whistle 2030b for user exhalation. The first portion having the greater inner diameter of each open tube whistle 2030b for user exhalation is provided in fluid communication and adjacent to the external side 2006 of the breathing mask 2002. The second portion having the smaller inner diameter of each open tube whistle 2030b for user exhalation is provided in fluid communication and adjacent to the internal side 2004 of the breathing mask 2002.

As can be seen in Figures 27a and 27b, the first portions of the open tube whistles 2030a, 2030b having the greater inner diameter are downstream (i.e. in the direction of fluid flow) from the second portions of the open tube whistle having the smaller inner diameter.

The generated audible/sound signal may provide feedback to a user about the user's breathing pattern, lung function condition, and/or any lung function abnormalities. For example, the audible/sound signal may be audible to the user, such that the user is provided with immediate feedback regarding their breathing rate. Alternatively/additionally, the audible/sound signal may be detectable by a computing device which may monitor a frequency (e.g. pitch), duration, and/or rate of audible/sound signals (e.g. number of sound signals per minute), and may provide feedback to the user (e.g. via a display of the computing device).

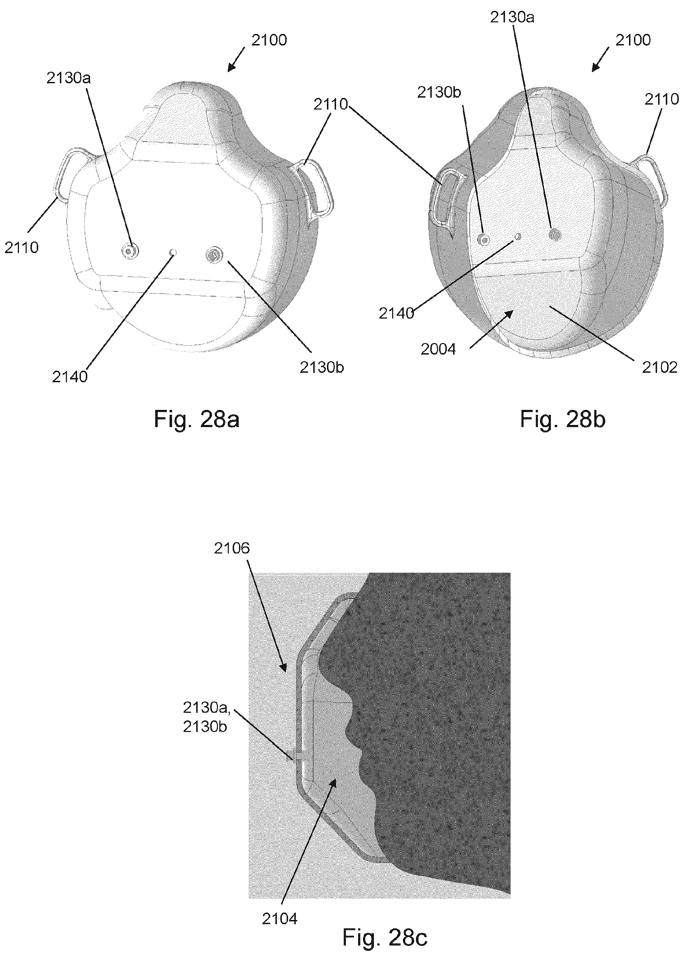

Figures 28a-28c show another oral mask assembly 2100. Oral mask assembly 2100 is similar to oral mask assembly 2000 shown in Figures 26a-26d and 27a and 27b, except for that oral mask assembly 2100 does not comprise one-way valves. However oral mask assembly 2100 does comprise a bypass channel 2140 that extends between the internal side 2104 of the breathing mask 2102 and the external side 2006 of the breathing mask 2102. The absence of the pair of one-way valves allows for a more compact oral mask assembly 2100, whilst helping to adjust the resistance of fluid flow through the oral mask assembly 2100.

Oral mask assembly 2100 comprises two open tube whistles 2130a, 2130b, a first open tube whistle 2130a for generating an audible/sound signal during user inhalation, and the other open tube whistle 2130b for generating an audible/sound signal during user exhalation. In particular, the open tube whistle 2130a for generating an audible/sound signal during user inhalation has a first portion with a greater inner diameter in fluid communication with, and adjacent to, the internal side 2004 of the breathing mask 2102, and a second portion with a smaller inner diameter in fluid communication with, and adjacent to, the external side 2006 of the breathing mask 2102. The open tube whistle 2130b for generating an audible/sound signal during user exhalation has a first portion with a greater inner diameter in fluid communication with, and adjacent to, the external side 2006 of the breathing mask 2102, and a second portion with a smaller inner diameter in fluid communication with, and adjacent to, the internal side 2004 of the breathing mask 2102.

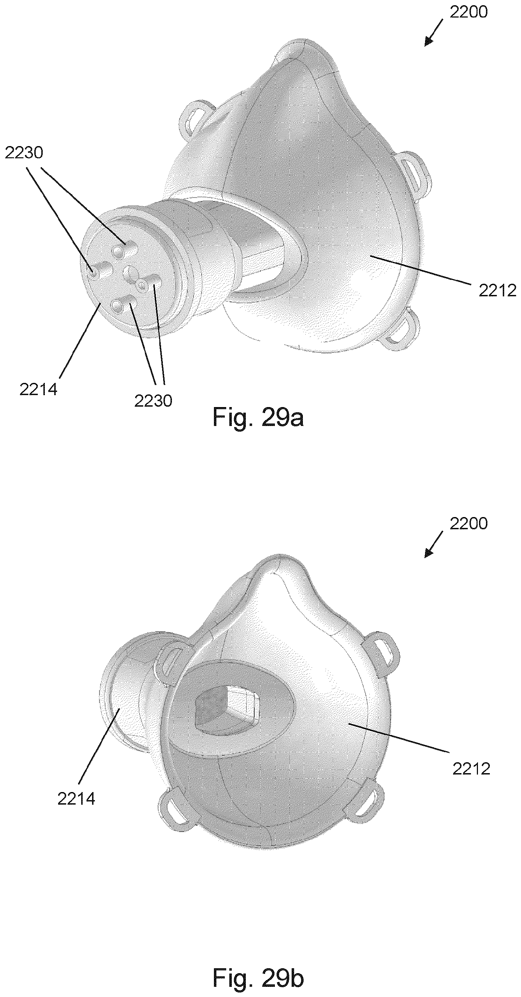

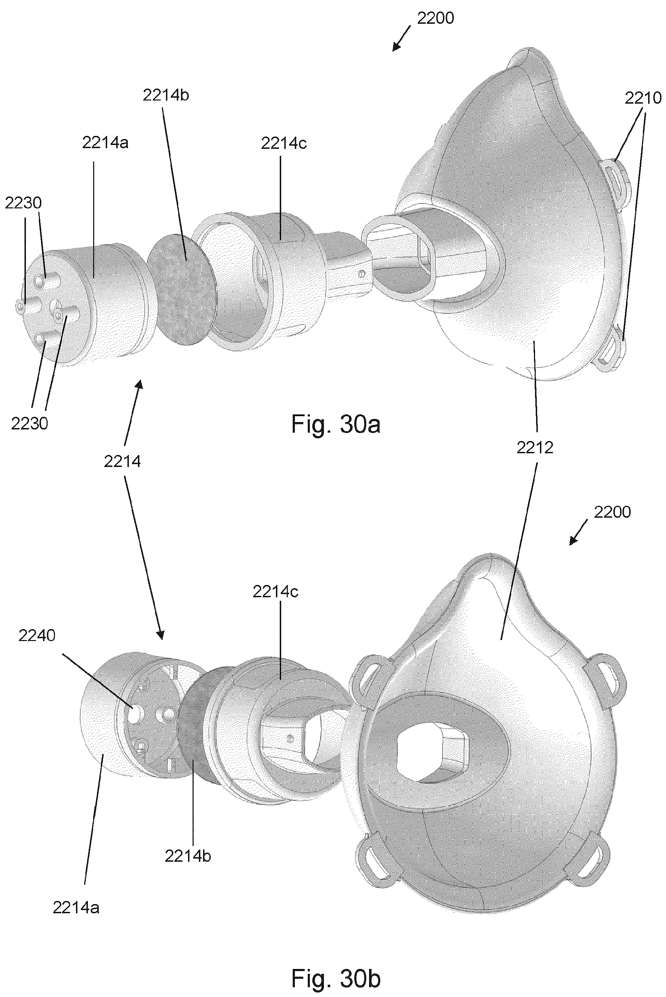

Figure 29a, 29b, 30a and 30b show an oral mask assembly 2200 which is similar to the oral mask assemblies 2000, 2100 described above, except that oral mask assembly 2200 comprises a main body 2212 and an add-on component 2214. The add-on component 2214 is attachable to the main body 2212, in particular within a recess of the main body 2212.

The add-on assembly 2214 comprises a plurality (in this case, four) open tube whistles 2230. Two of the open tube whistles are for generating an audible/sound signal during user inhalation, and the other two open tube whistles are for generating an audible/sound signal during user exhalation. The geometries and dimensions of the open tube whistles may be as described above in relation to oral mask assembly 2000 and oral mask assembly 2100.

Figures 30a and 30b show exploded views of oral mask assembly 2200. The add-on component 2214 comprises a plurality of sub-components, including a whistle sub-component 2214a, a filter 2214b and an attachment sub-component 2214c for attaching to the main body 2212. The whistle sub-component 2214a comprises the open tube whistles 2230 and a bypass channel 2240. The whistle sub-component 2214a and the attachment sub-component 2214c may be injection moulded from a polymer material such as PVC, ABS or PP.

The sub-components 2214a, 2214b, 2214c may be attachable together by one or more snap-fit or push-fit connections, by an interference fit or by one or more threaded connections. The add-on component 2214 (e.g. the attachment sub-component 2214c) may be attachable to the main body 2212 via a snap-fit, push-fit, interference-fit or threaded connection. The filter 2214b is positioned between the whistle sub-component 2214a and the attachment sub-component 2214c.

Providing the open tube whistles 2230 in a replaceable sub-component 2214 means that the open tube whistles can be replaced with open-tube whistles of different geometries and/or dimensions which may thus generate an audible/sound signal at a different predetermined rate of fluid flow. Thus, a single main body 2212 may be (re)usable with different combinations of open tube whistles, whereas one or more of the sub-components 2214a, 2214b, 2214c may be discarded and/or replaced after use.

While the disclosure includes exemplary embodiments described above, many equivalent modifications and variations will be apparent to those skilled in the art when given this disclosure. Accordingly, the exemplary embodiments set forth above are considered to be illustrative and not limiting. Various changes to the described embodiments may be made without departing from scope of the claims.

## Claims

1. An inhaler device (1) for indicating a desired fluid flow rate, the device comprising:
an open tube whistle (10); and
a mouthpiece (3) in fluid communication with an external opening of the inhaler device and a first end of the open tube whistle (10a), wherein an inner diameter of the open tube whistle has a stepped profile such that, upon user inhalation through the mouthpiece, an audible/sound signal is generated at a predetermined fluid flow rate through the open tube whistle, wherein:
the stepped profile of the inner diameter of the open tube whistle comprises only one step;
the step is closer to a second end (10b) of the open tube whistle than the first end of the open tube whistle; **characterized in that**
the inner diameter of a first portion of the open tube whistle adjacent to the first end of the open tube whistle is greater than the inner diameter of a second portion of the open tube whistle adjacent to the second end of the open tube whistle.

2. The inhaler device of claim 1, wherein the second end (10b) of the open tube whistle provides an additional external opening of the inhaler device.

3. The inhaler device of claim 2, wherein the second end of the open tube whistle, the first end of the open tube whistle, and an opening of the mouthpiece for communication with the mouth of the user are collinear.

4. The inhaler device of claim 2 or claim 3, wherein a side wall of the open tube whistle defines at least one lateral hole.

5. The inhaler device of any of claims 2-4, further comprising a stem block (4) for location of a drug reservoir (5), the drug reservoir being operable to deliver a dose of drug through a drug delivery output aperture (14) into the mouthpiece for inhalation by the user, wherein the open tube whistle extends through the stem block for fluid communication with the mouthpiece.

6. The inhaler device of claim 5, wherein the stem block and the open tube whistle are integrally formed, and optionally further comprising an upright main body (2) for housing the drug reservoir, wherein the upright main body, the stem block and the open tube whistle are integrally formed.

7. The inhaler device of claim 5, wherein the open tube whistle is reversibly and slidably receivable in the stem block.

8. The inhaler device of claim 7, further comprising an upright main body (2) for housing the drug reservoir, wherein the stem block is reversibly and slidably receivable in the upright main body, and the open tube whistle is configured to extend through the stem block to lock the stem block to the upright main body.

9. The inhaler device of claim 8, wherein the upright main body comprises a locating feature, and the stem block comprises a locating feature, the stem block locating feature complimenting the main body locating feature to correctly position the stem block in the upright main body, and optionally, wherein the stem block locating feature is a locating protrusion and the main body locating feature is a locating recess, the stem block locating protrusion being configured to interlock with the main body locating recess.

10. The inhaler device of claim 8 or claim 9, further comprising a stem block guider (140) for guiding the stem block along one or more protruding ribs (141) in the upright main body, and optionally wherein the stem block guider is integrally formed with the stem block.

11. The inhaler device of any of claims 7-10, wherein the open tube whistle is biased into an assembled position in which the open tube whistle extends through the stem block.

12. The inhaler device of any preceding claim, wherein the inhaler device is a spacer.

13. The inhaler device of any of claims 1-11, wherein:
the inhaler device is a pressurised metered dose inhaler (pMDI); or
the inhaler device is a drug delivery inhaler device, comprising a salbutamol/HFA152a formulation.

14. A system comprising an inhaler device according to any one of the previous claims, and a computing device having a sound receiver for detecting the audible/sound signal.

## Patentansprüche

1. Inhaliervorrichtung (1) zum Angeben einer gewünschten Fluidströmungsrate, wobei die Vorrichtung Folgendes umfasst:
eine offene Rohrpfeife (10); und
ein Mundstück (3), das in Fluidkommunikation mit einer äußeren Öffnung der Inhaliervorrichtung und einem ersten Ende der offenen Rohrpfeife (10a) steht, wobei ein Innendurchmesser der offenen Rohrpfeife ein Stufenprofil aufweist, sodass beim Inhalieren über das Mundstück durch einen Benutzer bei einer vorbestimmten Fluidströmungsrate über die offene Rohrpfeife ein hörbares/Klangsignal erzeugt wird, wobei:
das Stufenprofil des Innendurchmessers der offenen Rohrpfeife nur eine Stufe umfasst;
die Stufe näher bei einem zweiten Ende (10b) der offenen Rohrpfeife als beim ersten Ende der offenen Rohrpfeife angeordnet ist; **dadurch gekennzeichnet, dass**
der Innendurchmesser eines zum ersten Ende der offenen Rohrpfeife benachbarten ersten Abschnitts der offenen Rohrpfeife größer ist als der Innendurchmesser eines zum zweiten Ende der offenen Rohrpfeife benachbarten zweiten Abschnitts der offenen Rohrpfeife.

2. Inhaliervorrichtung nach Anspruch 1, wobei das zweite Ende (10b) der offenen Rohrpfeife eine zusätzliche äußere Öffnung der Inhaliervorrichtung bereitstellt.

3. Inhaliervorrichtung nach Anspruch 2, wobei das zweite Ende der offenen Rohrpfeife, das erste Ende der offenen Rohrpfeife und eine Öffnung des Mundstücks zur Kommunikation mit dem Mund des Benutzers kollinear sind.

4. Inhaliervorrichtung nach Anspruch 2 oder 3, wobei eine Seitenwand der offenen Rohrpfeife zumindest ein seitliches Loch definiert.

5. Inhaliervorrichtung nach einem der Ansprüche 2 bis 4, ferner umfassend einen Schaftblock (4) zum Anordnen eines Arzneimittelreservoirs (5), wobei das Arzneimittelreservoir betreibbar ist, um eine Arzneimitteldosis über eine Arzneimittelabgabeausgangsöffnung (14) in das Mundstück zum Inhalieren durch den Benutzer abzugeben, wobei die offene Rohrpfeife sich zur Fluidkommunikation mit dem Mundstück durch den Schaftblock hindurch erstreckt.

6. Inhaliervorrichtung nach Anspruch 5, wobei der Schaftblock und die offene Rohrpfeife einstückig ausgebildet sind, und gegebenenfalls ferner umfassend einen aufrechten Hauptkörper (2) zum Aufnehmen des Arzneimittelreservoirs, wobei der aufrechte Hauptkörper, der Schaftblock und die offene Rohrpfeife einstückig ausgebildet sind.

7. Inhaliervorrichtung nach Anspruch 5, wobei die offene Rohrpfeife umkehrbar und gleitend im Schaftblock aufnehmbar ist.

8. Inhaliervorrichtung nach Anspruch 7, ferner umfassend einen aufrechten Hauptkörper (2) zum Aufnehmen des Arzneimittelreservoirs, wobei der Schaftblock umkehrbar und gleitend im aufrechten Hauptkörper aufnehmbar ist, und wobei die offene Rohrpfeife dazu ausgelegt ist, sich durch den Schaftblock hindurch zu erstrecken, um den Schaftblock mit dem aufrechten Hauptkörper zu verriegeln.

9. Inhaliervorrichtung nach Anspruch 8, wobei der aufrechte Hauptkörper ein Anordnungsmerkmal umfasst und der Schaftblock ein Anordnungsmerkmal umfasst, wobei das Anordnungsmerkmal des Schaftblocks das Anordnungsmerkmal des Hauptkörpers ergänzt, um den Schaftblock korrekt im aufrechten Hauptkörper zu positionieren, und wobei gegebenenfalls das Anordnungsmerkmal des Schaftblocks ein Anordnungsvorsprung ist und das Anordnungsmerkmal des Hauptkörpers eine Anordnungsaussparung ist, wobei der Anordnungsvorsprung des Schaftblocks dazu ausgelegt ist, mit der Anordnungsaussparung des Hauptkörpers in Eingriff zu gelangen.

10. Inhaliervorrichtung nach Anspruch 8 oder 9, fermer umfassend ein Schaftblockführungselement (140) zum Führen des Schaftblocks entlang einer oder mehrerer hervorstehender Rippen (141) im aufrechten Hauptkörper, und wobei das Schaftblockführungselement gegebenenfalls mit dem Schaftblock einstückig ausgebildet ist.

11. Inhaliervorrichtung nach Anspruch 7 bis 10, wobei die offene Rohrpfeife in eine zusammengesetzte Position vorgespannt ist, in welcher sich die offene Rohrpfeife durch den Schaftblock hindurch erstreckt.

12. Inhaliervorrichtung nach einem der vorangegangenen Ansprüche, wobei die Inhaliervorrichtung ein Abstandselement ist.

13. Inhaliervorrichtung nach einem der Ansprüche 1 bis 11, wobei:
die Inhaliervorrichtung ein druckbeaufschlagtes Dosierinhaliergerät (pMDI) ist; oder
die Inhaliervorrichtung eine Arzneimittelabgabeinhaliervorrichtung ist, welche eine Salbutamol/HFA-152a-Formulierung umfasst.

14. System, umfassend eine Inhaliervorrichtung nach einem der vorangegangenen Ansprüche und eine Computervorrichtung, die einen Klangempfänger zum Detektieren des hörbaren/Klangsignals aufweist.

## Revendications

1. Dispositif d'inhalation (1) pour indiquer un débit d'écoulement de fluide souhaité, le dispositif comprenant :
un sifflet à tube ouvert (10) ; et
un embout buccal (3) en communication fluidique avec une ouverture externe du dispositif d'inhalation et une première extrémité du sifflet à tube ouvert (10a), dans lequel un diamètre interne du sifflet à tube ouvert présente un profil étagé tel que, lors d'une inhalation par l'utilisateur à travers l'embout buccal, un signal audible/sonore est généré à un débit d'écoulement de fluide prédéterminé à travers le sifflet à tube ouvert, dans lequel :
le profil étagé du diamètre intérieur du sifflet à tube ouvert ne comprend qu'un seul gradin ;
le gradin est plus proche d'une seconde extrémité (10b) du sifflet à tube ouvert que la première extrémité du sifflet à tube ouvert ; **caractérisé en ce que**
le diamètre intérieur d'une première partie du sifflet à tube ouvert adjacente à la première extrémité du sifflet à tube ouvert est supérieur au diamètre intérieur d'une seconde partie du sifflet à tube ouvert adjacente à la seconde extrémité du sifflet à tube ouvert.

2. Dispositif d'inhalation selon la revendication 1, dans lequel la seconde extrémité (10b) du sifflet à tube ouvert fournit une ouverture externe supplémentaire du dispositif d'inhalation.

3. Dispositif d'inhalation selon la revendication 2, dans lequel la seconde extrémité du sifflet à tube ouvert, la première extrémité du sifflet à tube ouvert, et une ouverture de l'embout buccal pour une communication avec la bouche de l'utilisateur sont colinéaires.

4. Dispositif d'inhalation selon la revendication 2 ou 3, dans lequel une paroi latérale du sifflet à tube ouvert définit au moins un trou latéral.

5. Dispositif d'inhalation selon l'une quelconque des revendications 2 à 4, comprenant en outre un bloc-tige (4) pour l'emplacement d'un réservoir de médicament (5), le réservoir de médicament pouvant fonctionner pour délivrer une dose de médicament à travers une ouverture de sortie de délivrance de médicament (14) dans l'embout buccal pour une inhalation par l'utilisateur, dans lequel le sifflet à tube ouvert s'étend à travers le bloc-tige pour une communication fluidique avec l'embout buccal.

6. Dispositif d'inhalation selon la revendication 5, dans lequel le bloc-tige et le sifflet à tube ouvert sont formés d'un seul tenant, et comprenant en outre facultativement un corps principal vertical (2) destiné à loger le réservoir de médicament, dans lequel le corps principal vertical, le bloc de tige et le sifflet à tube ouvert sont formés d'un seul tenant.

7. Dispositif d'inhalation selon la revendication 5, dans lequel le sifflet à tube ouvert peut être reçu de manière réversible et coulissante dans le bloc-tige.

8. Dispositif d'inhalation selon la revendication 7, comprenant en outre un corps principal vertical (2) destiné à loger le réservoir de médicament, dans lequel le bloc-tige peut être reçu de manière réversible et coulissante dans le corps principal vertical, et le sifflet à tube ouvert est configuré pour s'étendre à travers le bloc-tige pour verrouiller le bloc-tige au corps principal vertical.

9. Dispositif d'inhalation selon la revendication 8, dans lequel le corps principal vertical comprend une caractéristique de positionnement, et le bloc-tige comprend une caractéristique de positionnement, la caractéristique de positionnement de bloc-tige complétant la caractéristique de positionnement de corps principal pour positionner correctement le bloc-tige dans le corps principal vertical, et facultativement, dans lequel la caractéristique de positionnement de bloc-tige est une saillie de positionnement et la caractéristique de positionnement de corps principal est un évidement de positionnement, la saillie de positionnement de bloc-tige étant configurée pour se verrouiller mutuellement avec l'évidement de positionnement de corps principal.

10. Dispositif d'inhalation selon la revendication 8 ou la revendication 9, comprenant en outre un guide de bloc-tige (140) pour guider le bloc-tige le long d'une ou plusieurs nervures saillantes (141) dans le corps principal vertical, et facultativement dans lequel le guide de bloc-tige est formé d'un seul tenant avec le bloc-tige.

11. Dispositif d'inhalation selon l'une quelconque des revendications 7 à 10, dans lequel le sifflet à tube ouvert est sollicité dans une position assemblée dans laquelle le sifflet à tube ouvert s'étend à travers le bloc-tige.

12. Dispositif d'inhalation selon l'une quelconque des revendications précédentes, dans lequel le dispositif d'inhalation est un élément d'espacement.

13. Dispositif d'inhalation (170) selon l'une quelconque des revendications 1 à 11, dans lequel :
le dispositif d'inhalation est un inhalateur à dose mesurée mise sous pression (pMDI) ; ou
le dispositif d'inhalation est un dispositif d'inhalation d'administration de médicament, comprenant une formulation de salbutamol/HFA152a.

14. Système comprenant un dispositif inhalateur selon l'une quelconque des revendications précédentes, et un dispositif informatique présentant un récepteur de son pour détecter le signal sonore/audible.
